# EUROPEAN PATENT APPLICATION

(11) **EP 2 392 575 A1**
(43) Date of publication of application: **07.12.2011**
(21) Application number: 10164933.3
(22) Date of filing: 04.06.2010
(51) Int. Cl.: C07D 487/04

(54) **A novel synthetic approach to ß-aminobutyryl substituted compounds**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to process to prepare β-aminobutyryl compounds
of formula (I) 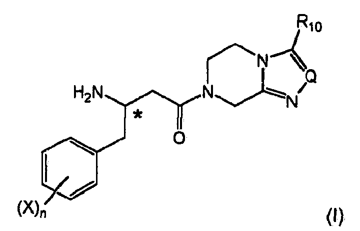 wherein Q is N, CH, C-CF₃ or C-phenyl, preferably N, and R₁₀ is H, C₁-C₄-alkyl or fluorinated C₁-C₂-alkyl, preferably trifluoromethyl,
and
wherein X is halogen selected from fluoro, chloro, or bromo, preferably fluoro, same or different, and n is 1-4.

## Description

### Field of the Invention

The present invention relates to the field of organic chemistry, more specifically to β-aminobutyryl substituted compounds, in particular β-aminobutyryl compounds having γ-aryl (notably aryl) groups, and/or heterocyclic structural moieties. Such compounds are useful as key structure framework of modern drug chemistry and especially of antidiabetic agents.

### Background of the Invention

β-Amino acids are of interest in the preparation of active pharmaceutical ingredients (APIs). The β-amino acid moiety in APIs of interest is normally part of a complex whole structure. Complexity is typically enhanced when considering a chiral center at the β-position of the β-aminobutyryl group and the general desire to obtain enantiopure compounds.

A particularly interesting class of APIs having β-amino acid structural moieties are dipeptidyl peptidase-4 (DPP-4) inhibitors which act as antidiabetic agents. DPP-4 inhibitors are oral antidiabetic drugs, which reduces glucose blood levels by a new mechanism of action in which the DPP - 4 inhibitors ("gliptins") inhibit inactivation of glucagon-like peptide (GLP), which stimulates insulin secretion. The benefit of these medicines lies in its lower side-effects (e.g., less hypoglycemia, less weight gain) in the control of blood glucose values. It can be used for treatment of diabetes mellitus type 2 either alone or in combination with other oral antihyperglycemic agents, such as metformin or a thiazolidinediones.

The first member of the novel pharmacological group is sitagliptin (compound of formula 1), which is chemically (*R*)-3-amino-1-(3-(trifluoromethyl)-5,6-dihydro-[1,2,4]triazolo[4,3-a]pyrazin-7(8H)-yl)-4-(2,4,5-trifluorophenyl)butan-1-one and which structure includes a β-amino acid part.

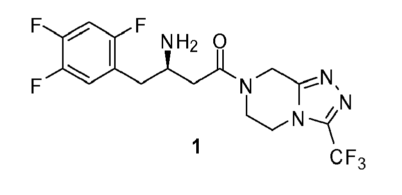

However, an inclusion of an β-amino acid framework into more complex molecules remains a permanent challenge for industrial production.

This is well reflected in the literature for the synthesis of sitagliptin. Several methods are described, how to introduce the β-amino acid structure into the molecule of sitagliptin. The first synthesis of sitagliptin molecule used chiral unusual dihydropyrazine chiral promoters, diazomethane and silver salts (WO 03/004498) which are unacceptable reagents for industrial synthesis - Scheme 1.

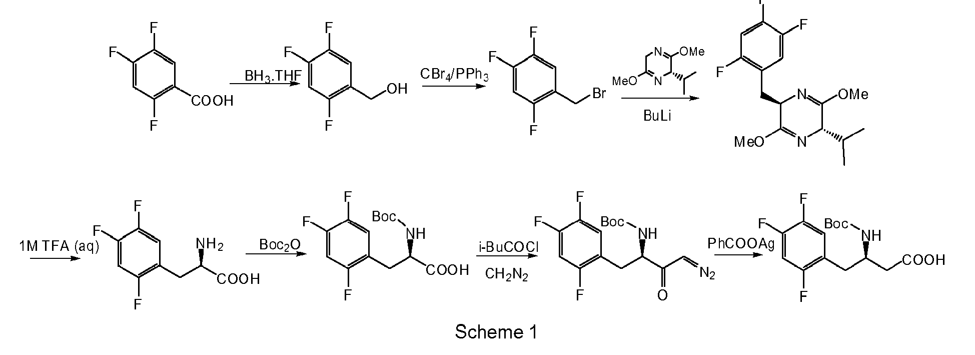

Better approaches include enantioselective hydrogenation of β-enamino acid derivatives but they need expensive precious metal catalysts, such as rhodium (WO 03/004498, Tetrahedron Asymmetry 17, 205 (2006)) or ruthenium (WO 09/064476) and expensive ligands, such as ferrocenyl diphosphine ligands - JOSIPHOS catalysts (WO 04/085378, WO 05/097733, WO 06/081151, J. Am. Chem. Soc., 126, 9918 (2004)).

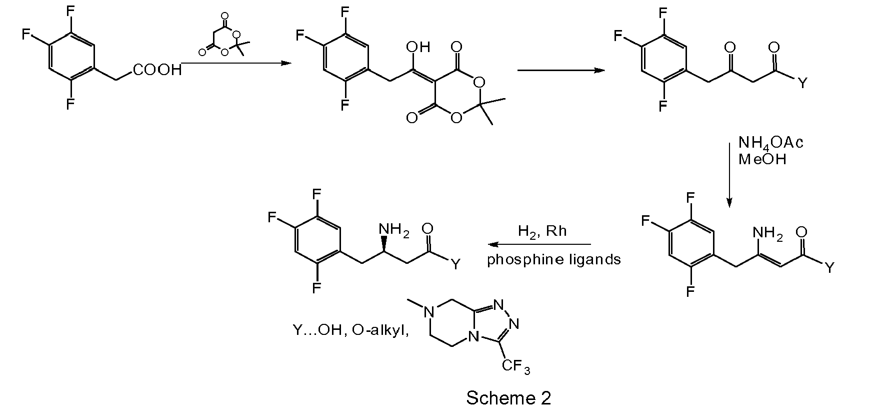

Another option is a hydrogenation with cheaper achiral catalyst, but with chiral derivatisation of enamines derived from phenylglycinamide - Scheme 3 (WO 04/085661). The obtained e.e. values are not sufficient for pharmaceutical use.

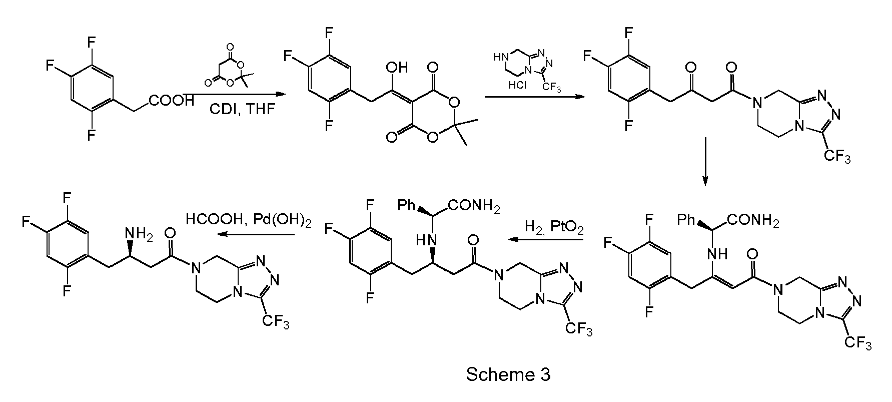

Yet another option is creating chiral centers by selective reduction of β-keto acid derivatives. Precious metal catalysts (WO 04/087650, Org. Prep. Res. & Dev. 9, 634-639 (2005)) or enzymatic reduction (WO 09/045507) can be used, while the transformation of the obtained chiral hydroxyl intermediates to final sitagliptin precursors via azetidinone intermediates is laborious - Scheme 4.

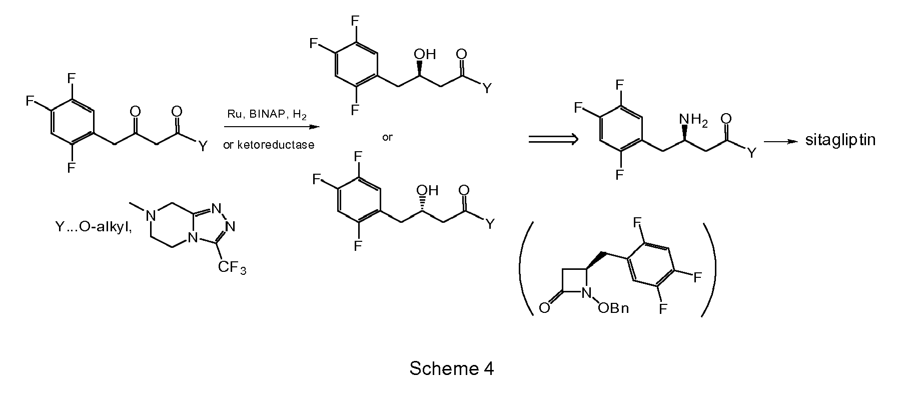

Most of described routes use 2,4,5-trifluorophenylacetic acid derivatives as starting materials which are prepared from 1-bromo-2,4,5-trifluorobenzene via organometal intermediates using copper (US 2004/068141), magnesium (US 2004/077901) and cobalt (CN 1749232) containing reagents. Organometals have been routinely used in industrial synthesis, but it is still more wished to avoid them, because their use requires more expensive special equipment.

Therefore, there is still a need for a simplification of industrial synthesis of β-amino acid derivatives as intermediates in the synthesis of dipeptidyl peptidase-4 (DPP-4) inhibitors such as sitagliptin.

### Summary of the invention

Aspects, advantageous features and preferred embodiments of the present invention summarized in the following items, respectively alone or in combination, contribute to solving this and other objects of the invention:
1. A process for the preparation of a β-amino amide compound, comprising:
   (i) providing a 2-nitropropionic acid derivative of formula (IIc) 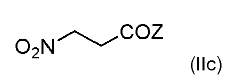 wherein Z is
      (i-1) a substituent defined by the following formula (III') 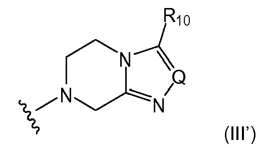 wherein Q is N, CH, C-CF₃, or C-phenyl, preferably N, and R₁₀ is H, C₁-C₄-alkyl or fluorinated C₁-C₂-alkyl, preferably trifluoromethyl,
         or
      (i-2) OH, OR¹, NR²R³ or OSiR⁴R⁵R⁶, wherein R¹ is C₁-C₆-alkyl or aryl-C₁-C₂-alkyl, R² is H, C₁-C₄-alkyl, arylmethyl, C₁-C₄-alkoxy or arylmethoxy, R³ is H, C₁-C₄-alkyl or arylmethyl and R⁴, R⁵ and R⁶ independently are C₁-C₄-alkyl or phenyl
   (ii) carrying out a coupling reaction (condensation) of the 2-nitropropionic acid derivative of formula (IIc) with an aldehyde of formula Ar-CHO, preferably in the presence of a base substance, wherein Ar denotes respectively unsubstituted or substituted phenyl, alkyl-aryl, alkoxy-aryl,
      and obtaining a compound of formula (VIIIa)or (VIa) 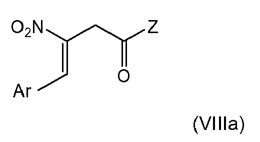 or 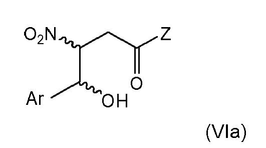
   (iii-1) subjecting (VIIIa) or (VIa) to a conversion reaction to obtain a compound of (IXa) 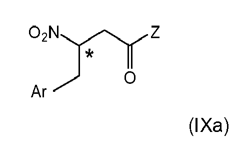 by a reaction selected from the group of esterification, dehydration, reduction, hydrogenation, elimination and combinations thereof,
      or
   (iii-2) subjecting (VIIIa) to a reduction reaction to obtain 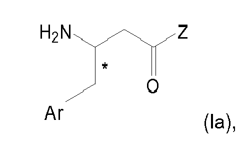 optionally forming a salt thereof;
      wherein the stereogenic center marked with an * is either in (*R*)-or (*S*)-configuration at marked center, or it is in racemic form,
   (iv) if Z is optionally defined as in (i-2), subjecting Z to structural modification, yielding a β-nitro amide compound; and
   (v) if the process optionally proceeds via (iii-1) or (iv), reducing the nitro group in compound (IXa) or the reaction products of (iv) to an amino group, optionally forming a salt thereof.
      The proceeding with compound (IIc) as defined in (i-1) leads to the advantage that pre-built structural moieties can be provided in high yield in the Z group and can efficiently be coupled with Ar-CHO. The procedural concept of the present invention further allows simple and efficient synthesis schemes starting from readily available or synthesizable starting compounds, as the compound of formula (VIa) provides for useful subsequent synthetic possibilities, and alternatively enables simple conversion to the structural form of formula (VIIIa) with further reaction options. The double bond substituents in formula (VIIIa) can be in a Z- and/or *E*-configuration, with its individual stereoisomers being separated into either *Z*- or *E*-configuration, or forming a *Z*-/*E*-mixture, allowing subsequent stereospecific reactions if desired.
      Moreover according to the basic procedural concept of the present invention, key structures are obtained which are particularly suitable for inclusion of an β-amino amide framework into more complex molecules. The process according to the present invention allows to obtain a range of β-aminobutyryl compounds, particular those including γ-aryl (especially phenylic) structural moieties in the form of "Ar" defined above, and/or variable structural moieties in the form of "Z" defined above or structurally modified forms, preferably heterocyclic groups. The process according to the present invention is particularly advantageous for producing the characteristic gliptin structure of dipeptidyl peptidase-4 (DPP-4) inhibitors unmet in the above discussed conventional synthetic strategies to obtain gliptins.
      In particular, by the process of the present invention synthetic routes are made possible without requiring protective groups and without requiring the use of exotic reagents. Moreover, if desired, stereogenic centers can be efficiently created and eventually resolved into respective stereoisomeric compounds, at least including a putative stereogenic center at the β-carbon atom of the β-amino acid structural framework, thereby enhancing industrial acceptance and suitability of the process of the present invention.
2. The process according to item 1, wherein the step of providing 2-nitropropionic amide derivative of formula (IIc) as defined in (i-1) comprises
   activating 3-nitropropionic acid of formula (II) 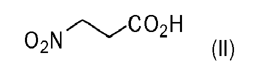 by conversion to
   (i) halogenide of formula (IIa) 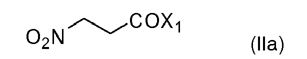 wherein X₁ is chloro, bromo, preferably chloro, by reaction with a sulfur, phosphorous, oxalyl or phosgene halogen derivative;
   (ii) or anhydride of formula (IIb) 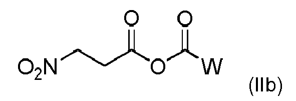 wherein W is selected from the group consisting of C₁-C₆-alkyl residues, C₁-C₆-alkoxy residues, unsubstituted or substituted benzyloxy, by reaction with acyl chlorides, preferably pivaloyl chloride, or chloroformates;
   (iii) or *in situ* formed intermediate by reaction with a coupling reagent known from peptide chemistry, selected from the group consisting of
      carbodiimides, preferably N,N'-dicyclohexylcarbodiimide (DCC), N,N'-diisopropylcarbodiimide (DPCI), or N-ethyl-N'-(3-dimethyaminopropyl) carbodiimide (EDC), with or without 1-hydroxybenzotriazole;
      activated ureas, preferably O-(benzotriazol-1-yl)-N,N,N',N'-tetramethyluronium hexafluorophosphate (HBTU), O-(benzotriazol-1-yl)-N,N,N',N'-tetramethyluronium tetrafluoroborate (TBTU), and O-(7-aza-benzotriazol-1-yl)-N,N,N',N'-tetramethyl uronium hexafluorophosphate (HATU);
      carbonyldiimidazole; and
      1-methylpyridinium iodide; and
   reacting the activated derivative of 3-nitropropanoic acid with the compound (III) 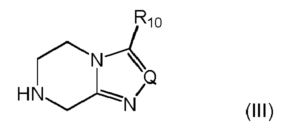 wherein Q and R₁₀ are as defined above.
   The activation step is suitably carried out in an aprotic solvent, preferably a solvent selected from halogenated aliphatic hydrocarbons, aromatic hydrocarbons, ethers and nitriles and mixture thereof, preferably acetonitrile.
3. The process according to item 1 or 2, wherein in a preferred embodiment of the present invention the substituent Z as defined under (i-1) has the following formula (IIIa') 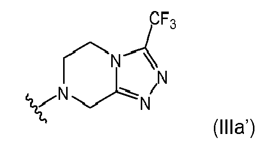 The preferred embodiment accordingly can beneficially be carried out by reacting an activated 3-nitropropionic acid of formula II according to item 2 with a compound of formula (IIIa) below: 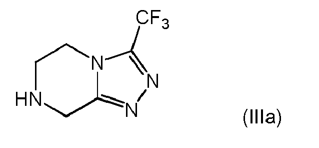 According to this preferred embodiment, already prepared heterocyclic groups are readily and efficiently coupled to a structural framework from which β-amino amide compounds and valuable intermediate compounds thereof can derive.
   The reaction with the compound of formula (IIIa) is suitably carried out in an aprotic solvent as described in item 2 above.
4. The process according to any one of the preceding items, wherein the aldehyde Ar-CHO is defined by formula (V) 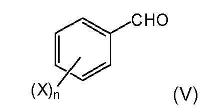 wherein X is halogen selected from fluoro, chloro, or bromo, preferably fluoro, same or different, and n is 1-4; in a specifically preferred embodiment the aldehyde Ar-CHO is defined by formula (Va) 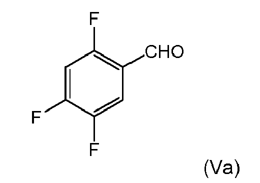 This embodiment can beneficially couple aromatic groups to a structural framework from which β-amino amide compounds and valuable intermediate compounds thereof can derive.
   The reaction is preferably carried out in the presence of a base substance. Suitable and preferable base substances in the process of the present invention are selected from the group of typically used bases in organic synthesis. The preferred base is selected from inorganic basic salts, such as acetates, carbonates, phosphates, most preferably potassium phosphate tribasic in submolar amounts and possibly even catalytic amounts, preferably in 5-20 % molar amount relative to compound (IIc).
   The reaction can be suitably carried out in a polar protic or an aprotic solvent, preferably selected from water, alcohols, amides, sulfoxides, sulphones or nitriles, preferably in acetonitrile.
   When combined with the specific embodiment of item 1, (i-1), a highly efficient and economic preparation of the compound of formula (VI) can preferably be achieved: 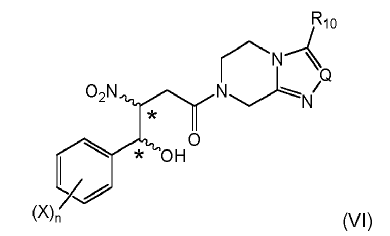 Stereogenic centers are marked with an *; these can be either in (*R*)- or (*S*)-configuration at marked center, and the reaction typically gives a mixture of stereoisomers. If desired, the stereoisomers can be resolved into the respective (*R*)- or (*S*)-isomers. X, n, Q and R₁₀ are the same as above.
5. The process according to any one of the preceding items, wherein the compound of formula (VIa) is obtained and subjected to a dehydration reaction by treating said compound with a strong acid or by using a dehydrating agent, respectively to give a compound of formula (VIIIa) 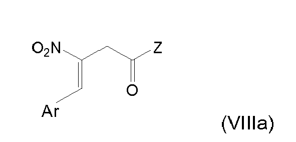 wherein Ar and *Z* are as defined above.
   The Ar- and nitro-groups at the double bond can be in a *Z*- and/or *E*-configuration, with its individual stereoisomers being separated, or forming a *Z*-/*E*-mixture. In a preferred embodiment of the present invention the Ar and the nitro group are in *E*-configuration.
   The strong acid may be selected from, without being limited to, hydrochloric acid, hydrobromic acid, sulfuric acid, methanesulfonic acid, phosphoric acid, polyphosphoric acid. The dehydrating agent may be selected from, without being limited to phosphorus pentoxide, phosphorus oxychloride, molecular sieves, dried aluminium oxide.
   When combined with the preferred embodiments of items 1, (i-1) and 4, a highly efficient and economic preparation a compound of formula (VIII) can be achieved according to a further preferred embodiment 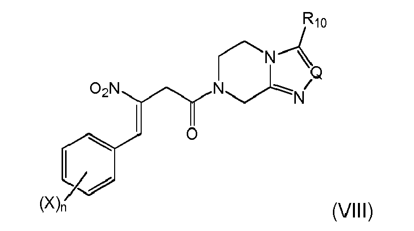 wherein X, n, Q and R₁₀ are as defined above.
6. The process according to any one of items 1 to 4, wherein the compound of formula (VIa) is subjected to a reduction reaction to reduce the benzylic hydroxyl group by treatment with silicon hydrides, or by catalytic hydrogenation in the presence of a precious metal catalyst, or by transformation to an ester and catalytic hydrogenation of the ester, respectively to give the compound (IXa) 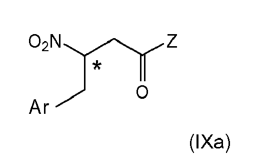 wherein the stereogenic center marked with an * is either in (*R*)- or (*S*)-configuration at marked center, or it is in racemic form; and Ar and Z are as defined above.
   When combined with the specific embodiments of items 1, (i-1) and 4, a highly efficient and economic preparation a compound of formula (IX) can be achieved according to a further preferred embodiment 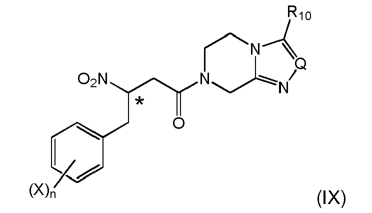 Suitable silicon hydrides for the reduction of the benzylic hydroxyl group in (VIa) include trialkylsilanes, wherein the alkyl group consists of more than one carbon atom and is preferably ethyl.
7. The process according to any one of the preceding items, wherein the compound of formula (VIa) is subjected to esterification reaction by halogenides or anhydrides of unsubstituted or halogenated C₁-C₆-alkanoic or C₁-C₆-alkanesulfonic acids, unsubstituted or para substituted benzenesulfonic acids, respectively in the presence of a base substance to give an ester compound of formula (VIIa) 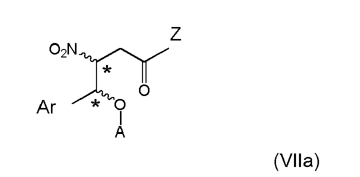 wherein stereogenic centers are marked with an *; these can be either in (*R*)- or (*S*)-configuration at marked center; Ar and Z are as defined above, and A is C₁-C₆-alkanoyl, halogenated C₁-C₆-alkanoyl, C₁-C₆-alkanesulfonyl, unsubstituted or para substituted benzenesulfonyl.
   The obtained acylated compound typically is a short-live intermediate. Beneficially it can be spontaneously converted, suitably under the same or similar reaction conditions of the present esterification step or by way of carrying out a heating step, to a subsequent product eliminating the A-O group as shown in the next item.
   When combined with the specific embodiments of items 1, (i-1) and 4, a highly efficient and economic preparation an ester of formula (VII) can be achieved according to a further embodiment 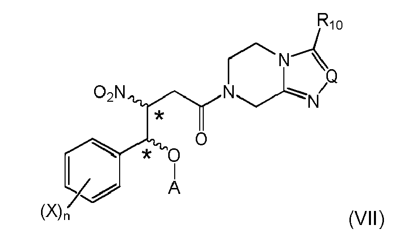 wherein stereogenic centers are marked with an *; these can be either in (*R*)- or (*S*)-configuration at marked center; and A, X, n, Q and R₁₀ are as defined above.
8. The process according to item 7, wherein the ester compound of formula (VIIa) is subjected to base-induced or thermal elimination of AOH from said compound to give a compound of formula (VIIIa) 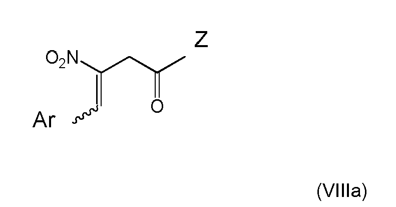 The Ar- and nitro-groups at the double bond can be in a Z- and/or *E*-configuration, with its individual stereoisomers being separated, or forming a Z-/*E*-mixture.
   When combined with the specific embodiments of items 1, (i-1) and 4, preferably a highly efficient and economic preparation a compound of formula (VIII) is achieved 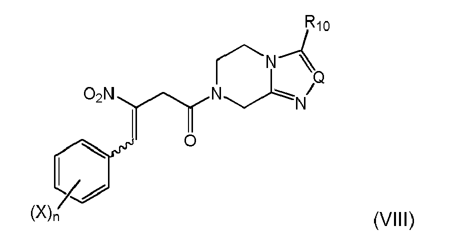 wherein X, n, Q and R¹⁰ are as defined above.
9. The process according to item 5 or 8, wherein the double bond of formula (VIIIa) is reduced by a reducing agent, by catalytic hydrogenation or by biotransformation, respectively to give a compound of formula (IXa) 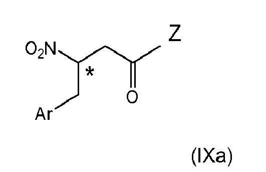 wherein the stereogenic center marked with an * is either in (*R*)- or (*S*)-configuration at marked center, or it is in racemic form, and Ar is as defined above.
   Suitable reducing agents include, without being limited to, boron hydrides and aluminium hydrides. When combined with the specific embodiments of items 1, (i-1) and 4, preferably a highly efficient and economic preparation a compound of formula (IX) is achieved 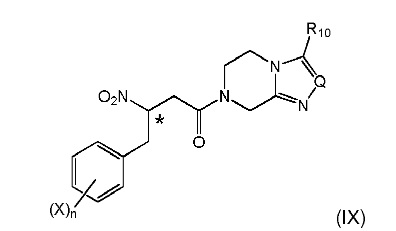 wherein the stereogenic center marked with an * is either in (*R*)- or (*S*)-configuration at marked center, or it is in racemic form, and X, n, Q and R₁₀ are as defined above.
10. The process according to any one of items 1 to 5 and 8, wherein the compound of formula (VIIIa) is subjected to a simultaneous reduction of the double bond and the nitro group by a suitable reduction system to give the compound of formula (Ia) 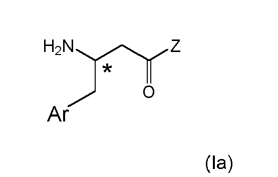 wherein the stereogenic center marked with an * is either in (*R*)- or (*S*)-configuration at marked center, or it is in racemic form, Ar and Z are as defined above. When combined with the preferred specific of items 1, (i-1) and 4, a particularly preferred, efficient and economic preparation a compound of formula (I) can be achieved 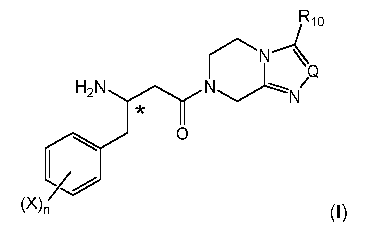
11. The process according to any one of items 1 to 9, wherein in item 1, (v) the nitro group is converted into an amino group by contacting with a reducing agent, preferably selected from elemental metals in the presence of acids, preferably zinc and tin, cations in low oxidation states selected from Fe(II), Sn(II), Cr(II), hydrogen sulfide, metal sulfides and polysulfides,
   or by catalytic hydrogenation in the presence of a metal catalyst, preferably selected from nickel, palladium, ruthenium, rhodium, iridium, gold, and platinum, to give the final compound of formula (I).
   When combined with the specific embodiment of item 6, preferably the preparation of a compound of formula (I) having included the β-amino amide part coupled to both aromatic and heterocylcle groups is achieved 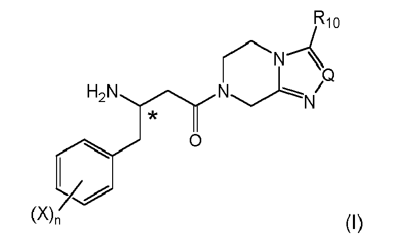 wherein the stereogenic center marked with an * is either in (*R*)- or (*S*)-configuration at marked center, or it is in racemic form, and X, n, Q and R₁₀ are as defined above.
   The various embodiments of items 1 and 4 to 11 can be summarized by the following scheme (Ar as defined above; Z being defined by (i); stereogenic centers are marked with an *; these can be either in (*R*)- or (*S*)-configuration at marked center, or it is in racemic form): 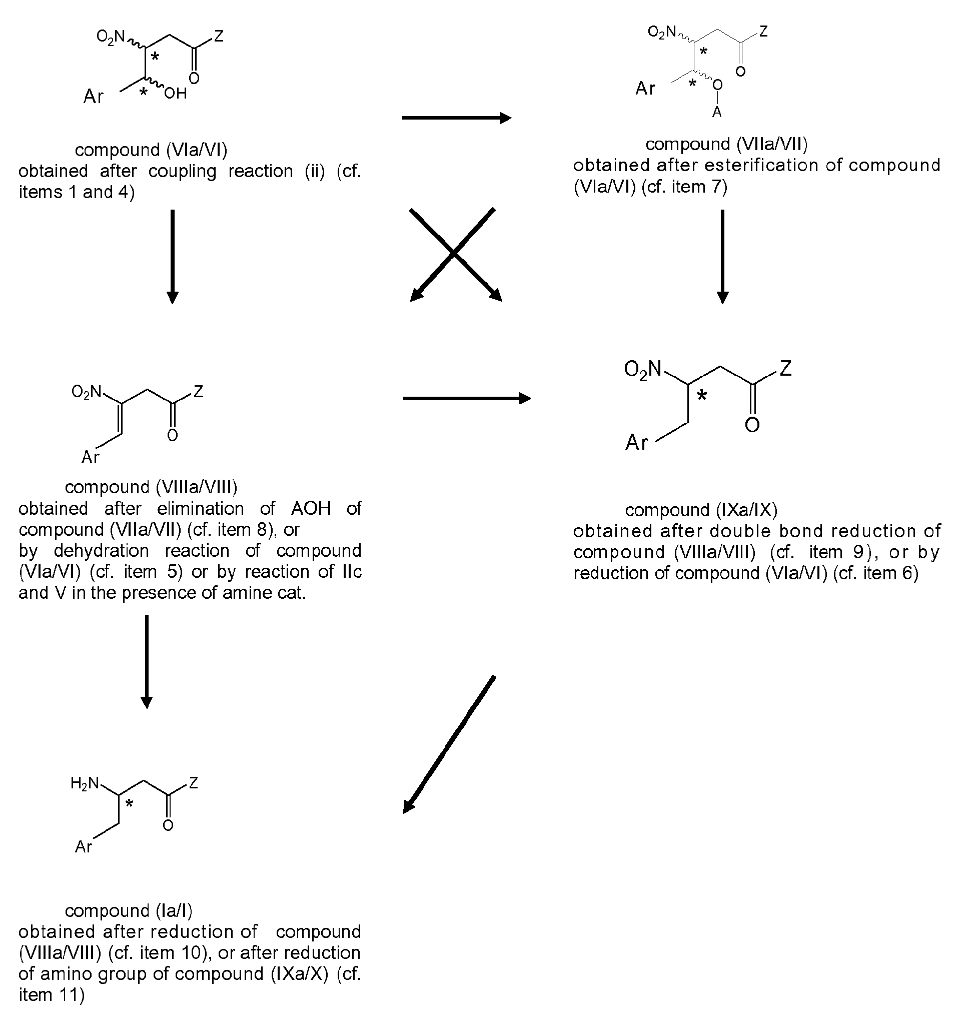
12. The process according to any one of items 1 and 4 to 11, wherein when the 2-nitropropionic acid derivative of formula (IIc) 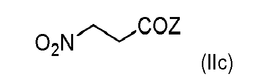 is provided with Z being defined as in item 1, (i-2)
   the coupling reaction (ii) with an aldehyde of formula (V) is carried out forming the intermediates (Xa) or (XIIa) 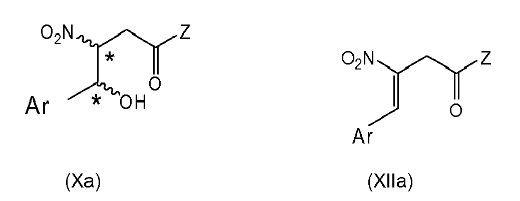 wherein Ar and * are as defined above,
   and wherein subsequently the intermediates of formula (Xa), or (XIIa) are subjected to the conversion (iii) to obtain a compound of formula (XIIIa), 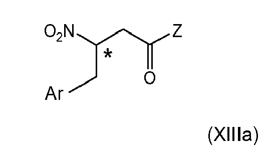 wherein (Xa), (XIIa) and (XIIIa) are respective structural analogues of (VIa), (VIIIa), and (IXa) in which Z is defined as in item 1, (i-2) and
   wherein the compound (XIIIa), if Z is other than OH, is converted by a method selected from the group consisting of hydrolysis, reduction and hydrogenation, to give the respective carboxylic compound formula (XIVa) 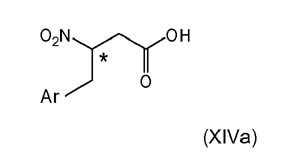 which is subjected to further structural modification.
   This alternative of proceeding with compound (IIc) with a Z group as defined herein provides the advantage that carboxy, ester or amide groups subsequently can be readily subjected to structural modifications, e.g. as described in item 1, (iv) or (v), to eventually further built-up desirable structural moieties.
   Ar is as defined above. More preferably, Ar is 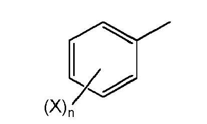 Consequently in preferred embodiments of item 12, compounds listed by the following formulae can be effectively obtained in preferred embodiments, respectively (Z being as defined here in item 12): 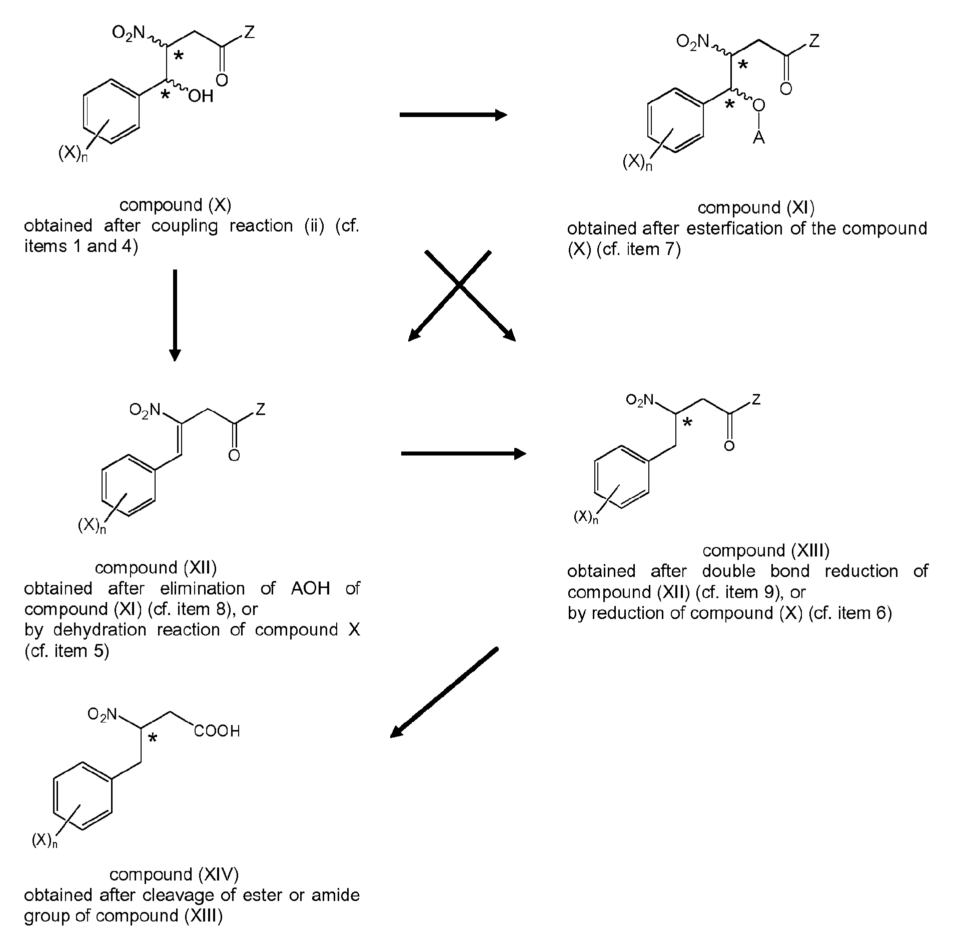 In the compound formulae shown above, stereogenic centers are marked with an *; these can be either in (*R*)- or (*S*)-configuration at marked center, or it is in racemic form.
   In alternative embodiments, the compound of formula (X) can be dehydrated or the compound of formula (XI) can eliminate AOH, which depending on conditions can occur spontaneously, optionally after additional heating, to obtain the compound of formula (XII) (cf. items 7 and 8). As another alternative, the compound of formula (X) is subjected to dehydration reaction (cf. item 5), to give the compound of formula (XII).
   According to another embodiment, the compound of formula (X) is subjected to reduction reaction (cf. item 6), or the compound of formula (XII) is subjected to double bond reduction (cf. item 9), respectively to give the compound of formula (XIII). The compound of formula (XIII) then only needs to be subjected to cleavage reaction of the ester or amide bond present in the *Z* group.
13. The process according to item 12, wherein subjecting compound (XIV) to further structural modification involves converting the carboxylic group to an activated carboxylic group by
   (i) reaction with a sulfur, phosphorous, oxalyl or phosgene halogen derivative to obtain respective halogenide COX₁, wherein X₁ is chloro, bromo, preferably chloro; or
   (ii) reaction with acyl chlorides, preferably pivaloyl chloride, or chloroformates to obtain an anhydride wherein Z is defined below 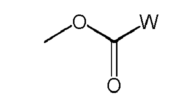 wherein W is C₁-C₆-alkyl or C₁-C₆-alkoxy, unsubstituted or substituted benzyloxy; or
   (iii) by reaction with coupling reagents which are ordinary used in peptide chemistry to obtain *in situ* formed intermediates, wherein the coupling reagents are selected from the group consisting of:
      carbodiimides, preferably from N,N'-dicyclohexylcarbodiimide (DCCI), N,N'-diisopropylcarbodiimide (DPCI), or N-ethyl-N'-(3-dimethyaminopropyl) carbodiimide (EDC), with or without 1-hydroxybenztriazole,
      activated ureas preferably O-(benzotriazol-1-yl)-N,N,N',N'-tetramethyluronium hexafluorophosphate (HBTU), O-(benzotriazol-1-yl)-N,N,N',N'-tetramethyluronium tetrafluoroborate (TBTU), and O-(7-aza-benzotriazol-1-yl)-N,N,N',N'-tetramethyl uronium hexafluorophosphate (HATU)
      carbonyldiimidazole,
      1-methylpyridinium iodide.
      When Ar preferably is 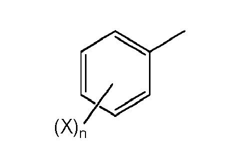 in the preferred embodiments of item 13, compounds listed by the following formulae (XIVa) and (XIVb) can be effectively obtained in preferred embodiments, respectively: 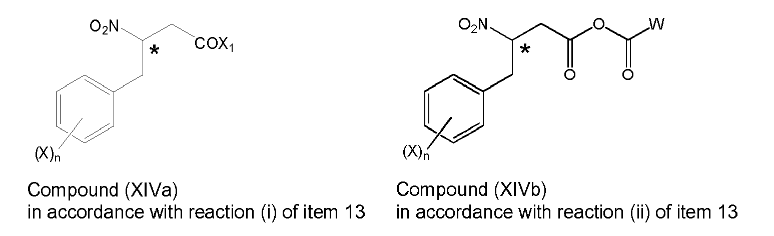 wherein the stereogenic center marked with an * is either in (*R*)- or (*S*)-configuration at marked center, or it is in racemic form; and X₁ and W is as defined above.
14. The process according to item 13, wherein the activated compound obtained in either one of (i), (ii) or (iii) is reacted with a compound of formula (III) 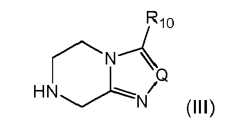 wherein Q is N, CH, C-CF₃, or C-phenyl, preferably N, and R₁₀ is H, C₁-C₄-alkyl or fluorinated C₁-C₂-alkyl, preferably trifluoromethyl to give a compound of formula (IXb) 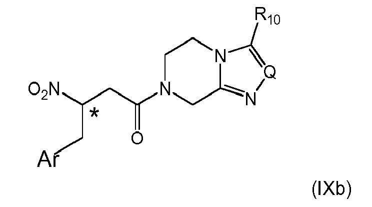 wherein the stereogenic center marked with an * is either in (*R*)- or (*S*)-configuration at marked center, or it is in racemic form; and Ar is as defined above;
   and subsequently the nitro group is reduced to amino group, preferably as defined in item 11.
   When preferably Ar is 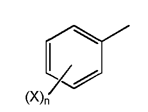 the compound of formula IX can be efficiently obtained 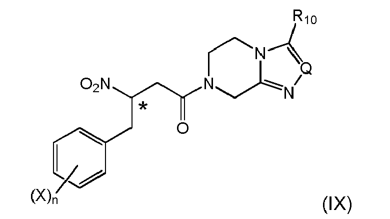 wherein the stereogenic center marked with an * is either in (*R*)- or (*S*)-configuration at marked center, or it is in racemic form, and X, n, Q and R₁₀ are as defined above.
   And after reduction of the amino group, the compound of formula (I) can be obtained.
15. The process according to any one of items 1 to 6 and 9 to 14, wherein the β-γ double bond saturation or reduction is respectively carried out in enantioselective manner and enriching either the (R)- or the (S)-enantiomer, preferably the (R)-enantiomer is enriched.
   According to this preferred embodiment, the following (R)-enantiomeric stereogenic center is achieved, respectively shown for intermediate nitro compounds as well as for the reduced amino compounds 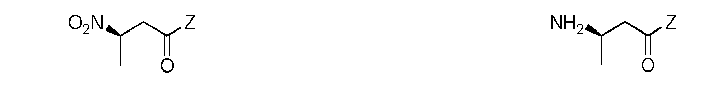 wherein Z and the remaining structure is as defined above.
16. The process according to any one of items 1 to 6 and 9 to 14, wherein the compound of formula (VIIIa/VIII) is subjected to hydrogenation in the presence of a transition metal and at least one chiral ligand to give a compound of formula (IXa) 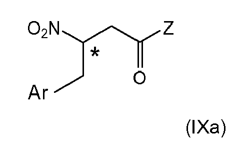 wherein Ar and Z are as defined above,
   wherein the configuration on stereogenic center marked with an * is enriched in (*R*)- or (*S*)-configuration, preferably is enriched in (*R*)-enantiomer.
   When Ar is as preferably defined above the compound of formula (IX) can be efficiently obtained in preferred enantiomeric excess (e.e). values as described below.
   The transition metal is preferably selected from nickel, palladium, ruthenium, rhodium, iridium, gold and platinum. The chiral ligand is selected from phosphines, diphosphines, phosphates, phosphites, phosphoroamidites aminoalcohols, diamines, amine-amides, amine-phosphine ligand, or combination thereof such as BINAP, DAIPEN and QUINAP.
17. The process according to any one of items 1 to 6 and 9 to 14, wherein the compound of formula (VIIIa) is subjected to reduction by a reductase, selected from reductase enzymes of various microorganisms, preferably selected from reductase enzymes derived from Actinomyces, Saccharomyces (in particular baker's yeast) or Bacteria gens Clostridium.
18. The process according to any one of the preceding items, wherein any of the specified enantiomers at the respective stereogenic center * at the γ-carbon atom is enriched as the (*R*)-enantiomer or the (*S*)-enantiomer, preferably as the (*R*)-enantiomer.
   As the method of enantiomer enrichment, any one of the embodiments defined in items 15 to 17 can preferably be used, and/or other conventional ways for enantiomeric enrichment such as chromatographic techniques may be used alternatively or in addition, such as chiral matrix chromatography.
   In this manner the (*R*)-enantiomer or the (*S*)-enantiomer can be respectively and specifically enriched to over 30 % e.e., more preferably to over 60 % e.e., most preferably to over 90 % e.e.
   Most preferable, the compound of formula (IX) is enriched as (*R*)-enantiomer, preferably to over 60 % e.e., more preferably to over 90 % e.e., most preferably to over 97 % e.e.
   The preferred embodiments defined in items are industrially acceptable and efficient processes to beneficial prepare DPP-4 inhibitors in high enantiomeric purity.
19. The process according to any one of items 1 to 18 used for the preparation of sitagliptin, wherein the substituents in formulas (I) to (XIII) have the following meaning
   n is 3
   X is fluoro in positions 2, 4 and 5 of benzene ring as regards to the side chain
   Q is N
   R₁₀ is trifluoromethyl.
   It has thus been demonstrated that the process of the present invention allows to obtain dipeptidyl peptidase-4 (DPP-4) inhibitors in an industrially applicable, economical and acceptable manner.
   Preferably, it is readily possible to yield the compound of formula (X) to be in enantiomerically pure form, more preferably is in the (*R*)-configuration at marked center. Specifically, sitagliptin (compound of formula 1) is obtained in a highly efficient process.
20. A compound defined by the following formula (VI) 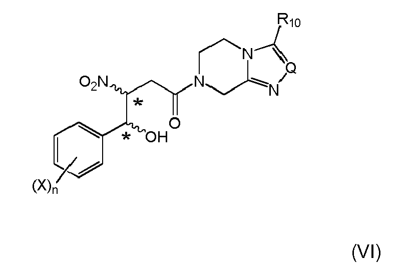 wherein the stereogenic centers marked with an * are either in (*R*)- or (*S*)-configuration at marked center; X is halogen selected from fluoro, chloro, or bromo, preferably fluoro, same or different, and n is 1-4, preferably 3; Q is N, CH, C-CF₃, or C-phenyl, preferably N; and R₁₀ is H, C₁-C₄-alkyl or fluorinated C₁-C₂-alkyl, preferably trifluoromethyl.
21. A compound defined by the following formula 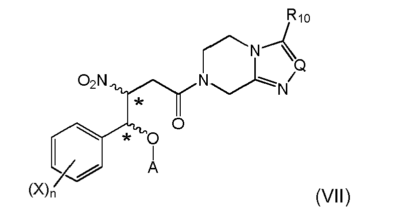 wherein the stereogenic centers marked with an * are either in (*R*)- or (*S*)-configuration at marked center; X is halogen selected from fluoro, chloro, or bromo, preferably fluoro, same or different, and n is 1-4, preferably 3; A is C₁-C₆-alkanoyl, halogenated C₁-C₆-alkanoyl, C₁-C₆-alkanesulfonyl, unsubstituted or para substituted benzenesulfonyl; Q is N, CH, C-CF₃, or C-phenyl, preferably N; and R₁₀ is H, C₁-C₄-alkyl or fluorinated C₁-C₂-alkyl, preferably trifluoromethyl.
22. A compound defined by the following formula (VIII) 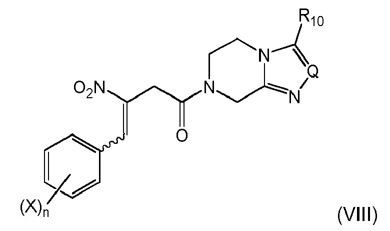 wherein X is halogen selected from fluoro, chloro, or bromo, preferably fluoro, same or different, and n is 1-4, preferably 3; Q is N, CH, C-CF₃, or C-phenyl, preferably N; and R₁₀ is H, C₁-C₄-alkyl or fluorinated C₁-C₂-alkyl, preferably trifluoromethyl.
23. A compound defined by the following formula (IX) 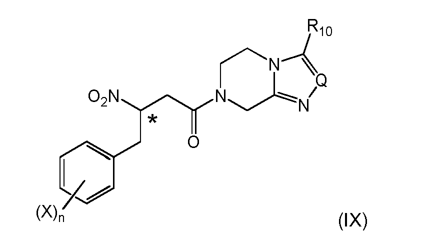 wherein the stereogenic center marked with an * is either in (*R*)- or (*S*)-configuration at marked center, or it is in racemic form, preferably in (*R*)- configuration; X is halogen selected from fluoro, chloro, or bromo, preferably fluoro, same or different, and n is 1-4, preferably 3; Q is N, CH, C-CF₃, or C-phenyl, preferably N; and R₁₀ is H, C₁-C₄-alkyl or fluorinated C₁-C₂-alkyl, preferably trifluoromethyl.
24. 4-Hydroxy-3-nitro-1-(3-(trifluoromethyl)-5,6-dihydro-[1,2,4]triazolo[4,3-a]pyrazin-7(8H)-yl)-4-(2,4,5-trifluorophenyl)butan-1-one of the following formula: 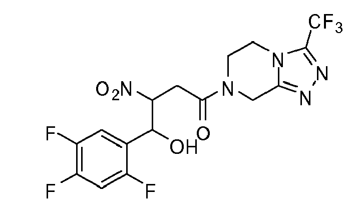
25. 3-Nitro-1-(3-(trifluoromethyl)-5,6-dihydro-[1,2,4]triazolo[4,3-a]pyrazin-7(8H)-yl)-4-(2,4,5-trifluorophenyl)but-3-en-1-one: 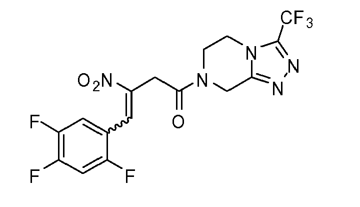
26. 3-Nitro-1-(3-(trifluoromethyl)-5,6-dihydro-[1,2,4]triazolo[4,3-a]pyrazin-7(8H)-yl)-4-(2,4,5-trifluorophenyl)butan-1-one: 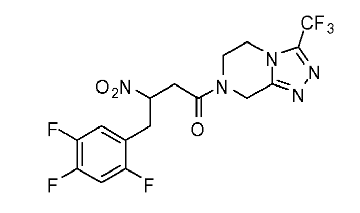
27. 3-(*R*)-Nitro-1-(3-(trifluoromethyl)-5,6-dihydro-[1,2,4]triazolo[4,3-a]pyrazin-7(8H)-yl)-4-(2,4,5-trifluorophenyl)butan-1-one: 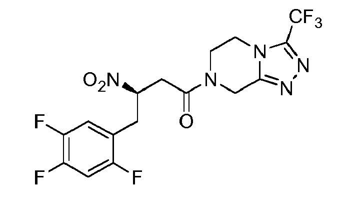 The compounds defined in items 20 to 27 respectively specify key intermediates useful for synthesizing gliptins (dipeptidyl peptidase-4 (DPP-4) inhibitors) as particular β-aminobutyryl compounds having γ-phenyl and/or heterocyclic structural moieties. As to preferably enantiomerically enriched compounds, reference is made to item 18 above.
28. Use of a compound as defined in any one of items 20 to 27 for the manufacture of gliptins (dipeptidyl peptidase-4 (DPP-4) inhibitors), preferably sitagliptin.
29. Process for the preparation of a pharmaceutical composition comprising a gliptin (dipeptidyl peptidase-4 (DPP-4) inhibitor), comprising:
   carrying out a process according to any one of items 1 to 19 and obtaining a gliptin compound;
   optionally transforming said gliptin compound into its pharmaceutically acceptable salt
   formulating said gliptin compound or its pharmaceutically acceptable salt with at least one pharmaceutical excipient.
30. The process according to item 29, wherein said gliptin is sitagliptin.
31. The process according to item 28, wherein said gliptin compound, preferably sitagliptin, is transformed into its phosphate salt.
32. The process according to any one of items 29 to 31, wherein said pharmaceutical composition comprises a combination of said gliptin compound and one or more additional pharmaceutically active ingredient(s), preferably selected from the group consisting of insulin sensitizers, insulin, insulin mimetics, sulfonylureas, α-glucosidase inhibitors, glucagon receptor antagonists, GLP-1, GLP-1 analogues, GLP-1 mimetics, GLP-1 receptor agonists, GIP, GIP mimetics, PACAP, PACAP mimetics, PACAP receptor agonists, cholesterol lowering agents, PPARδ agonists, antiobesity compounds, ileal bile acid tranporter inhibitors, agents intended for use in inflammatory conditions, antihypertensive agents, glucokinase activators (GKAs), inhibitors of 11β-hydroxysteroid dehydrogenase type 1, inhibitors of cholesteryl ester transfer protein (CETP) and inhibitors of fructose 1,6-bisphosphatase.
33. The process according to item 31, wherein said additional pharmaceutically active ingredient is metformin or its pharmaceutically acceptable salt.

### Detailed description of the invention

The present invention provides an industrially applicable, economical and acceptable preparation of β-aminobutyryl substituted compounds, in particular β-aminobutyryl compounds having γ-phenyl and/or heterocyclic structural moieties, which thereby leads to useful key structure framework of modern drug chemistry and especially of antidiabetic agents dipeptidyl peptidase-4 (DPP-4) inhibitors such as sitagliptin. The preparation process start from 3-nitroproprionic acid or derivatives thereof, and can preferably use 2,4,5-trifluorobenzaldehyde in the advantageous embodiment to prepare gliptin and related structures, as respective cheap commercial starting materials. 2,4,5-trifluorobenzaldehyde as the further starting compound for the preparation of gliptins is a product of classical fluoro chemistry without the need for organometals for its preparation. β-nitro propionic acid and its substituted forms and derivatives are easily available from corresponding acrylic derivatives. Moreover, the process of the present invention provides excellent options to introduce chiral centers at the β-carbon atom of the β-amino acid derivatives.

α-Hydroxy-β-amino butyric acid derivatives can be prepared by a condensation reaction of an α-hydroxy-β-nitro propionic acid and an aldehyde and subsequent reduction of the nitro group using Pd/C or Pd, respectively as hydrogenation catalyst. (JP7165678 (A), Chin. J. Med. Chem., 13, 5, 270). However, α-hydroxy-β-nitro propionic acid are no suitable precusors for the synthesis of gliptins and in particular of sitagliptin.

The term "alkyl" as used herein, if not stated otherwise with respect to particular embodiments, includes reference to a straight or branched chain alkyl moiety having 1, 2, 3, 4, 5 or 6 carbon atoms. This term includes methyl, ethyl, propyl (n-propyl or isopropyl), butyl (n-butyl, sec-butyl or tert-butyl), pentyl, hexyl and the like. In particular, alkyl may have 1, 2, 3 or 4 carbon atoms.

The term "cycloalkyl" as used herein, if not stated otherwise with respect to particular embodiments, includes an alicyclic moiety having 3, 4, 5, 6, 7 or 8 carbon atoms. The group may be a bridged or polycyclic ring system. More often cycloalkyl groups are monocyclic. This term includes groups such as cyclopropyl, cyclobutyl, cyclopentyl, cyclohexyl, norbomyl, bicyclo[2.2.2]octyl and the like.

The term "alkoxy" as used herein, if not stated otherwise with respect to particular embodiments, include -O-alkyl, wherein alkyl is straight or branched chain and comprises 1, 2, 3, 4, 5 or 6 carbon atoms. In certain embodiments, alkoxy has 1, 2, 3 or 4 carbon atoms. This term includes methoxy, ethoxy, propoxy, isopropoxy, butoxy, tert-butoxy, pentoxy, hexoxy and the like.

The term "aryl" as used herein, if not stated otherwise with respect to particular embodiments, includes reference to an aromatic ring system comprising 6, 7, 8, 9, 10, 11, 12, 13, 14, 15 or 16 ring carbon atoms. Aryl is often phenyl but may be a polycyclic ring system, having two or more rings, at least one of which is aromatic. This term includes phenyl, naphthyl, fluorenyl, azulenyl, indenyl, anthryl and the like.

The term "heterocycle" as used herein includes, if not stated otherwise with respect to particular embodiments, a saturated (e.g. heterocycloalkyl) or unsaturated (e.g. heteroaryl) heterocyclic ring moiety having 3, 4, 5, 6, 7, 8, 9, 10, 11, 12, 13, 14, 15 or 16 ring atoms, at least one of which is selected from nitrogen and oxygen. In particular, heterocyclyl includes a 3- to 10-membered ring or ring system and more particularly a 5- or 6-or 7-membered ring, which may be saturated or unsaturated; examples thereof include oxiranyl, azirinyl, 1,2-oxathiolanyl, imidazolyl, thienyl, furyl, tetrahydrofuryl, pyranyl, thiopyranyl, thianthrenyl, isobenzofuranyl, benzofuranyl, chromenyl, 2H-pyrrolyl, pyrrolyl, pyrrolinyl, pyrrolidinyl, imidazolyl, imidazolidinyl, benzimidazolyl, pyrazolyl, pyrazinyl, pyrazolidinyl, thiazolyl, isothiazolyl, dithiazolyl, oxazolyl, isoxazolyl, pyridyl, pyrazinyl, pyrimidinyl, piperidyl, piperazinyl, pyridazinyl, morpholinyl, thiomorpholinyl, especially thiomorpholino, indolizinyl, isoindolyl, 3H-indolyl, indolyl, benzimidazolyl, cumaryl, indazolyl, triazolyl, tetrazolyl, purinyl, 4H-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, ß-carbolinyl, phenanthridinyl, acridinyl, perimidinyl, phenanthrolinyl, furazanyl, phenazinyl, phenothiazinyl, phenoxazinyl, ehromenyl, isochromanyl, chromanyl and the like.

More specifically, a saturated heterocyclic moiety may have 3, 4, 5, 6 or 7 ring carbon atoms and 1, 2, 3, 4 or 5 ring heteroatoms selected from nitrogen and oxygen. The group may be a polycyclic ring system but more often is monocyclic, for example including azetidinyl, pyrrolidinyl, tetrahydrofuranyl, piperidinyl, oxiranyl, pyrazolidinyl, imidazolyl, indolizidinyl, piperazinyl, thiazolidinyl, morpholinyl, thiomorpholinyl, quinolinidinyl and the like. Furthermore, the "heteroaryl" may include an aromatic heterocyclic ring system having 5, 6, 7, 8, 9, 10, 11, 12, 13, 14, 15 or 16 ring atoms, at least one of which is selected from nitrogen and oxygen. The group may be a polycyclic ring system, having two or more rings, at least one of which is aromatic, but is more often monocyclic. This term includes pyrimidinyl, furanyl, benzo[b]thiophenyl, thiophenyl, pyrrolyl, imidazolyl, pyrrolidinyl, pyridinyl, benzo[b]furanyl, pyrazinyl, purinyl, indolyl, benzimidazolyl, quinolinyl, phenothiazinyl, triazinyl, phthalazinyl, 2H-chromenyl, oxazolyl, isoxazolyl, thiazolyl, isoindolyl, indazolyl, purinyl, isoquinolinyl, quinazolinyl, pteridinyl and the like.

The term "substituted" as used herein in reference to a structure/moiety/group means that one or more, especially up to 5, more especially 1, 2 or 3, of the hydrogen atoms in said structure/moiety/group are replaced independently of each other by the corresponding number of substituents known to a person skilled in the art. Typical substituents include, without being limited to halogen, trifluoromethyl, cyano, nitro, oxo, NR', -OR', -C(O)R', -C(O)OR', -OC(O)R', - S(O)R', N(R')R", C(O)N(R')R", -SO₂N(R')R' and R"', wherein each of R', R" and R"' are selected from the group consisting of C₁ - C₆ alkyl, C₁ - C₆ alkoxy, -(CH₂)ₘ-heterocyclyl (m being 1, 2, 4 or 4) and each R' and R" may be optionally and independently further substituted with one or more of hydrogen, halogen, cyano, amino, hydroxy, C₁ - C₆ alkyl and C₁ - C₆ alkoxy. Specific substituents in particular include halogen such as fluoro, chloro and/or bromo, hydroxy, amino, C₁ - C₆ alkyl and C₁ - C₆ alkoxy, and halogenated C₁ - C₆ alkyl and C₁ - C₆ alkoxy such as trifluoromethyl. It will be understood that substituents are at positions where they are chemically possible, it being known or evident to the person skilled in the art to decide (either experimentally or theoretically) without inappropriate effort whether a particular substitution is possible. For example, substituents which may be unstable or may affect reactions disclosed herein may be omitted, at least at the relevant stage of intermediate compound or of the affected reaction.

The term "base substance" used herein according to preferred embodiments can be any base known and typically used in organic synthesis. The base can include, without being limited to, amides, hydrides, hydroxides, amidines, tertiary amines. The preferred base is selected from inorganic basic salts, such as acetates, carbonates, phosphates, most preferably potassium phosphate tribasic in submolar amounts and possibly even catalytic amounts, preferably in 5-20 % molar amount relative to compound (IIc).

According to a preferred option (A) of an embodiment of the present invention for the synthesis of dipeptidyl peptidase-4 (DPP-4) inhibitors of formula (I) 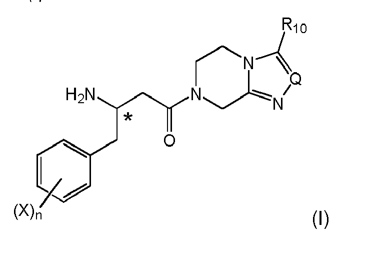 wherein the stereogenic center marked with an * is either in (*R*)- or (*S*)-configuration at marked center, or it is in racemic form, X is halogen selected from fluoro, chloro or bromo, preferably fluoro, same or different, n is 1-4, Q is N, CH, C-CF₃, or C-phenyl, preferably N, and R₁₀ is H, C₁-C₄-alkyl or fluorinated C₁-C₂-alkyl, preferably trifluoromethyl comprises
(a) activating 3-nitropropionic acid of formula (II) 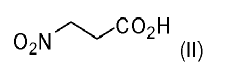 by conversion to
   (i) halogenide of formula (IIa) 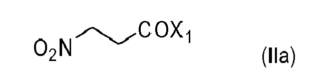 wherein X₁ is chloro, bromo, preferably chloro, by reaction with a sulfur, phosphorous, oxalyl or phosgene halogen derivative,
   (ii) anhydride of formula (IIb) 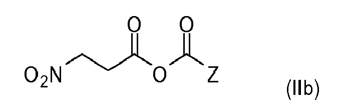 wherein Z is C₁-C₆-alkyl or C₁-C₆-alkoxy, unsubstituted or substituted benzyloxy with acyl chlorides, preferably pivaloyl chloride, or chlorofomate,
   (iii) in situ formed intermediates by coupling reagents which are ordinary used in peptide chemisty, selected from
      carbodiimides, preferably from N,N'-dicyclohexylcarbodiimide (DCC), N,N'-diisopropylcarbodiimide (DPCI), or N-ethyl-N'-(3-dimethyaminopropyl) carbodiimide (EDC), with or without 1-hydroxybenzotriazole,
      activated ureas preferably O-(benzotriazol-1-yl)-N,N,N',N'-tetramethyluronium hexafluorophosphate (HBTU), O-(benzotriazol-1-yl)-N,N,N',N'-tetramethyl uronium tetrafluoroborate (TBTU), and O-(7-aza-benzotriazol-1-yl)-N,N,N',N'-tetramethyl uronium hexafluorophosphate (HATU)
      carbonyldiimidazole,
      1-methylpyridinium iodide.
(b) reaction of the activated derivative of 3-nitropropanoic acid with a compound of formula (III) 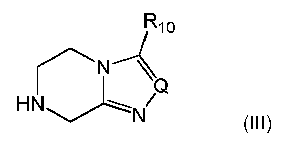 Q is N, CH, C-CF₃, or C-phenyl, preferably N, and R₁₀ is H, C₁-C₄-alkyl or fluorinated C₁-C₂-alkyl, preferably trifluoromethyl to give a compound of formula (IV) 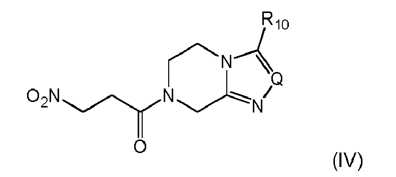 wherein Q and R₁₀ are as described above
(c) condensation of the compound of formula (IV) with an aromatic aldehyde of formula (V) 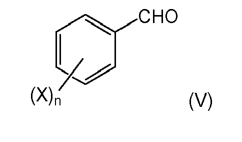 wherein X is halogen selected from fluoro, chloro, or bromo, preferably fluoro, same or different, and n is 1-4, in the presence of bases to give a mixture of compounds of formula (VI) 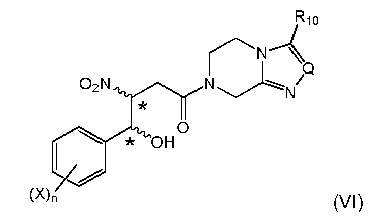 wherein the stereogenic centers marked with an * are either in (*R*)- or (*S*)-configuration at marked center, and X, n, Q and R₁₀ are the same as above
(d) esterification of the compound of formula (VI) by halogenides or anhydrides of unsubstituted or halogenated C₁-C₆-alkanoic or C₁-C₆-alkanesulfonic acids, unsubstituted or para substituted benzenesulfonic acids in the presence of bases to give an ester of formula (VII) 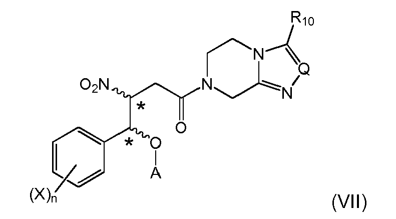 wherein A is C₁-C₆-alkanoyl, halogenated C₁-C₆-alkanoyl, C₁-C₆-alkanesulfonyl, unsubstituted or para substituted benzenesulfonyl, and X, n, Q and R₁₀ are the same as above, which normally is a short-live intermediate and can spontaneously be submitted to conversion of step (e) in reaction conditions of step (d)
(e) spontaneous, base induced or thermal, elimination of AOH from the compound of formula (VII) to give a compound of formula (VIII) 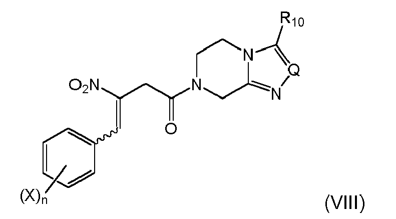 wherein and X, n, Q and R₁₀ are the same as above,
(f) saturation of the double bond by reducing agents, such as boron or aluminium hydrides, by catalytic hydrogenation or biotransformation to give a compound of formula (IX) 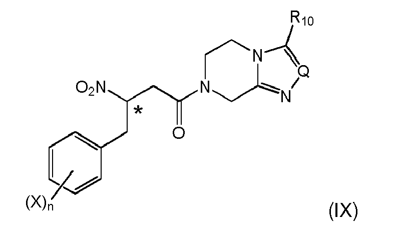 wherein the stereogenic center marked with an * is either in (*R*)- or (*S*)-configuration at marked center, or it is in racemic form, and X, n, Q and R₁₀ are the same as above,
(g) reduction of nitro group
   by reducing agents preferably selected from elemental metals in the presence of acids, preferably zinc and tin, cations in low oxidation states selected from Fe(II), Sn(II), Cr(II), hydrogen sulfide, metal sulfides and polysulfides, or
   by catalytic hydrogenation in the presence of nickel, palladium, platinum catalysts to give the final compound of formula (I).
   According to another option (B) of the embodiment present invention for the synthesis of dipeptidyl peptidase-4 (DPP-4) inhibitors, the compound of formula (VI) of the step (c) is dehydrated spontaneously in the conditions of the step (c) or after additional heating to give the compound of formula (VIII), so steps (d) and (e) are not necessary and are subsequently omitted.
   In another optional embodiment (C) steps (d) and (e) of the option (A) are replaced by
(h) dehydration reaction in which compound of formula (VI) 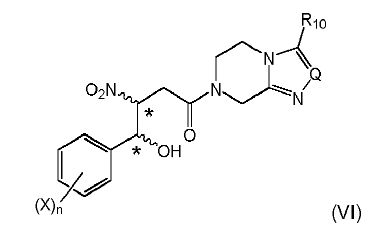 wherein the stereogenic centers marked with an * are either in (R)- or (*S*)-configuration at marked center, and X, n, Q and R₁₀ are the same as above
   is treated with a strong acid such as hydrochloric acid, hydrobromic acid, sulfuric acid, methanesulfonic acid, phosphoric acid, polyphosphoric acid or by using a dehydrating agent selected but not limited to phosphorus pentoxide, phosphorus oxychloride, molecular sieves, dried aluminium oxide to give the compound of formula (VIII) 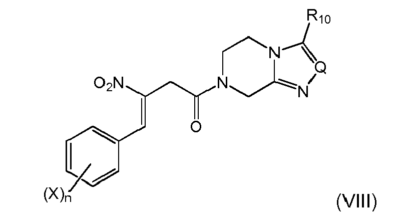 wherein and X, n, Q and R₁₀ are the same as above.
   In another optional embodiment (D) steps (d), (e) and (f) of the option (A) are replaced by
(i) reduction of the benzylic hydroxy group of compound of formula (VI) 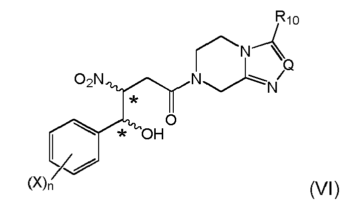 wherein the stereogenic centers marked with an * are either in (*R*)- or (*S*)-configuration at marked center, and X, n, Q and R₁₀ are the same as above,
   by treating with silicon hydrides such as triethylsilane, or by catalytic hydrogenation on precious metal catalyst, or by transformation of the compound (VI) to ester (VII) according to step (d) and catalytic hydrogenation of the ester to give the compound (IX) 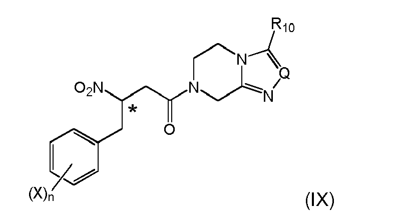 wherein the stereogenic center marked with an * is either in (*R*)- or (*S*)-configuration at marked center, or it is in racemic form, and X, n, Q and R₁₀ are the same as above.
   In another optional embodiment (E) steps (f) and (g) of the option (A) are replaced by
(j) simultaneous reduction of double bond and nitro group in which the compound of formula (VIII) 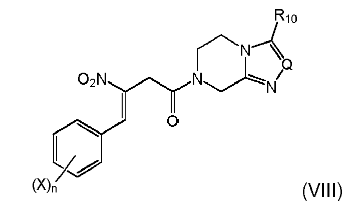 wherein and X, n, Q and R₁₀ are the same as above,
   is treated with a reducing agent, such as lithium aluminium hydride, sodium borohydride or boron trifluoride or by hydrogenation on metal catalysts to give the compound of formula (I) 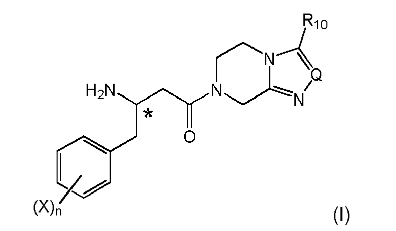 wherein the stereogenic center marked with an * is either in (*R*)- or (*S*)-configuration at marked center, X, n, Q and R₁₀ are the same as above.
   In another optional embodiment (F) steps (a) - (f) of the option (A) are replaced by the following set of steps
(k) condensation of the nitropropionic derivative of formula (IIc) 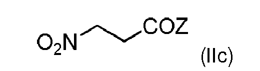 wherein Z is OH, OR¹, NR²R³ or OSiR⁴R⁵R⁶, wherein R¹ is C₁-C₆-alkyl or aryl-C₁-C₂-alkyl, R² is H, C₁-C₄-alkyl, arylmethyl, C₁-C₄-alkoxy or arylmethoxy, R³ is H, C₁-C₄-alkyl or arylmethyl and R⁴, R⁵ and R⁶ independently are C₁-C₄-alkyl or phenyl with the aldehyde of formula (V) 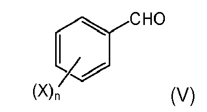 wherein X is halogen selected from fluoro, chloro, or bromo, preferably fluoro, same or different, and n is 1-4, in the presence of bases to give a mixture of compounds of formula (X) 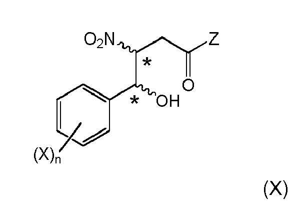 wherein the stereogenic centers marked with an * are either in (*R*)- or (*S*)-configuration at marked center, and X, n and Z are the same as above,
(l) esterification of the compound of formula (X) by halogenides or anhydrides of unsubstituted or halogenated C₁-C₆-alkanoic or C₁-C₆-alkanesulfonic acids, unsubstituted or para substituted benzenesulfonic acids in the presence of bases to give an ester of formula (XI) 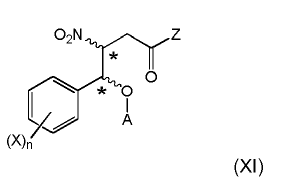 wherein A is C₁-C₆-alkanoyl, halogenated C₁-C₆-alkanoyl, C₁-C₆-alkanesulfonyl, unsubstituted or para substituted benzenesulfonyl, and A, X, n and Z are the same as above, which is in most cases a short-live intermediate, spontaneously submitted to conversion of step (m) in reaction conditions of step (I)
(m) spontaneous, base induced, or thermal elimination of AOH to give a compound of formula (XII) 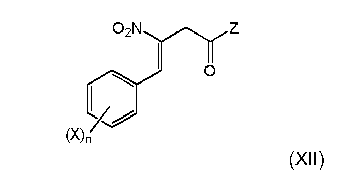 wherein and X, n and Z are the same as above,
(n) saturation of the double bond by reducing agents, such as boron or aluminium hydrides, by catalytic hydrogenation or biotransformation to give a compound of formula (XIII) 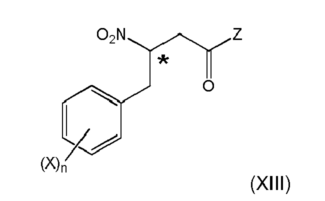 wherein the stereogenic center marked with an * is either in (*R*)- or (*S*)-configuration at marked center, or it is in racemic form, and X, n and Z are the same as above,
(o) if Z is other than OH cleavage of the ester or amid group of the compound of formula (XIII), wherein Z is OR¹, NR²R³ or OSiR⁴R⁵R⁶, wherein R¹ is C₁-C₆-alkyl or aryl-C₁-C₂-alkyl, R² is H, C₁-C₄-alkyl, arylmethyl, C₁-C₄-alkoxy or arylmethoxy, R³ is H, C₁-C₄-alkyl or arylmethyl and R⁴, R⁵ and R⁶ independently are C₁-C₄-alkyl or phenyl by conventional methods of hydrolysis, reduction or hydrogenation to give the compound of formula (XIV) 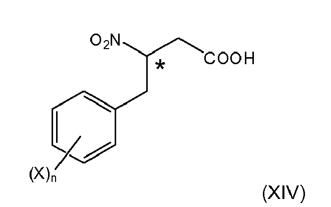 wherein X and n are the same as above
(p) conversion the compound of formula (XIV) to
   (i) halogenide of formula (XIVa) 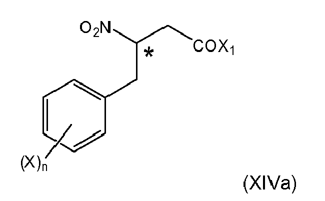 wherein X₁ is chloro, bromo, preferably chloro, by reaction with a sulfur, phosphorous, oxalyl or phosgene halogen derivative,
   (ii) anhydride of formula (XIVb) 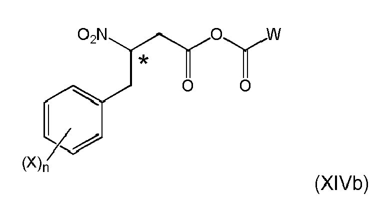 wherein W is C₁-C₆-alkyl or C₁-C₆-alkoxy, unsubstituted or substituted benzyloxy with acyl chlorides, preferably pivaloyl chloride, or chloroformates,
   (iii) *in situ* formed intermediates by coupling reagents which are ordinary used in peptide chemistry, selected from
      carbodiimides, preferably from N,N'-dicyclohexylcarbodiimide (DCC), N,N'-diisopropylcarbodiimide (DPCI), or N-ethyl-N'-(3-dimethyaminopropyl) carbodiimide (EDC), with or without 1-hydroxybenztriazole,
      activated ureas preferably O-(benzotriazol-1-yl)-N,N,N',N'-tetramethyluronium hexafluorophosphate (HBTU), O-(benzotriazol-1-yl)-N,N,N',N'-tetramethyluronium tetrafluoroborate (TBTU), and O-(7-aza-benzotriazol-1-yl)-N,N,N',N'-tetramethyluronium hexafluorophosphate (HATU)
      carbonyldiimidazole,
      1-methylpyridinium iodide.
(q) reaction of the activated derivative of the acid of formula (XIV) with a compound of formula (V) 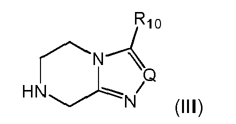 Q is N, CH, C-CF₃, or C-phenyl, preferably N, and R₁₀ is H, C₁-C₄-alkyl or fluorinated C₁-C₂-alkyl, preferably trifluoromethyl to give a compound of formula (IX) 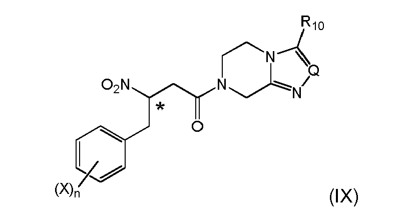 wherein the stereogenic center marked with an * is either in (*R*)- or (*S*)-configuration at marked center, or it is in racemic form, and X, n, Q and R₁₀ are the same as above,
   and the obtained compound is further treated according to the step (g) of the option (A).
   In the option (G) of the present invention the compound of formula (X) of the step (k) of option (F) is dehydrated spontaneously in the conditions of the step (k) or after additional heating to give the compound of formula (XII), so steps (I) and (m) are not necessary and are subsequently omitted.
   In another optional embodiment (H) steps (I) and (m) of the option (F) are replaced by
(r) dehydration reaction in which compound of formula (X) 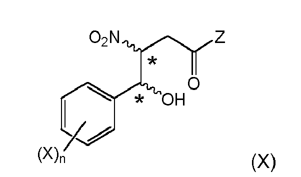 wherein the stereogenic centers marked with an * are either in (*R*)- or (*S*)-configuration at marked center, and X, n and Z are the same as above,
   is treated with strong acid such as hydrochloric acid, hydrobromic acid, sulfuric acid, methanesulfonic acid, phosphoric acid, polyphosphoric acid or by using a dehydrating agent selected but not limited to phosphorus pentoxide, phosphorus oxychloride, molecular sieves, dried aluminium oxide to give the compound of formula (XII) 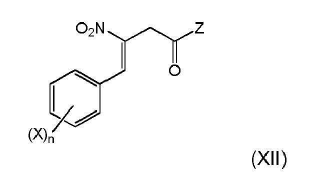 wherein and X, n, and Z are the same as above.
   In another optional embodiment steps (I), (m) and (n) of the option (F) are replaced by
(s) reduction of the benzylic hydroxy group of compound of formula (X) 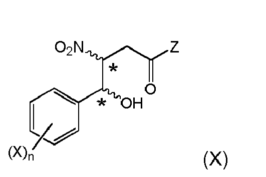 wherein the stereogenic centers marked with an * are either in (*R*)- or (*S*)-configuration at marked center, and X, n and Z are the same as above,
   by treating with silicon hydrides such as triethylsilane, or by catalytic hydrogenation on precious metal catalyst, or by transformation of the compound (X) to ester (XI) according to step (I) and catalytic hydrogenation of the ester to give the compound (XIII) 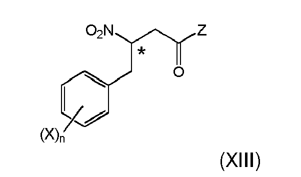 wherein the stereogenic center marked with an * is either in (R)- or (S)-configuration at marked center, or it is in racemic form, and X, n, and Z are the same as above.

In a special embodiment of the option (A) the saturation of the double bond (step (f)) is carried out in a chiral environment. Thus, the compound of formula (VIII) 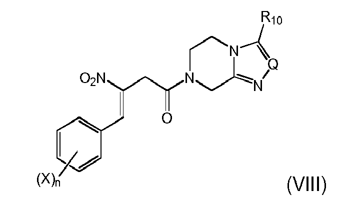 wherein X is halogen selected from fluoro, chloro or bromo, preferably fluoro, same or different, n is 1-4, Q is N, CH, C-CF₃, or C-phenyl, preferably N, and R₁₀ is H, C₁-C₄-alkyl or fluorinated C₁-C₂-alkyl, preferably trifluoromethyl, is treated by hydrogenation in the presence of transition metal preferably selected from ruthenium and in the presence of chiral ligands selected from phosphines, diphosphines such as BINAP to give a compound of formula (IX) 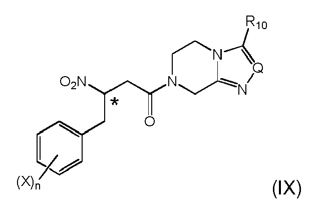 wherein X, n, Q and R₁₀ are the same as above and the configuration on stereogenic center marked with an * is enriched in (*R*)- or (*S*)-configuration, preferably is enriched over 30 % e. e., more preferably to over 60 % e. e., most preferably to over 90 % e. e.

In another option of enantioselective double bond saturation the compound of formula (VIII) can be subjected to biological reduction by reductases of various microorganisms preferably selected from the group Actinomyces, Saccharomyces (using baker's yeast) or Bacteria gens Clostridium.

In a preferred option for preparation of DPP-4 inhibitors the compound of formula (IX) is enriched in (*R*)-enantiomer more preferably to over 60 % e. e., more preferably to over 90 % e. e. most preferably to over 97 % e. e.

In a special embodiment of the invention the method is used for the preparation of sitagliptin. In a preferred embodiment the option (A) is used and the substituents in formulas (I) to (IX) have the following meaning
n is 3
X is fluoro in positions 2, 4 and 5 of benzene ring as regards to the side chain
Y is N
R is trifluoromethyl.

An illustrative and preferred but not limited approach of this special embodiment is shown in Scheme 5 and described in further detail as a representative embodiment below.

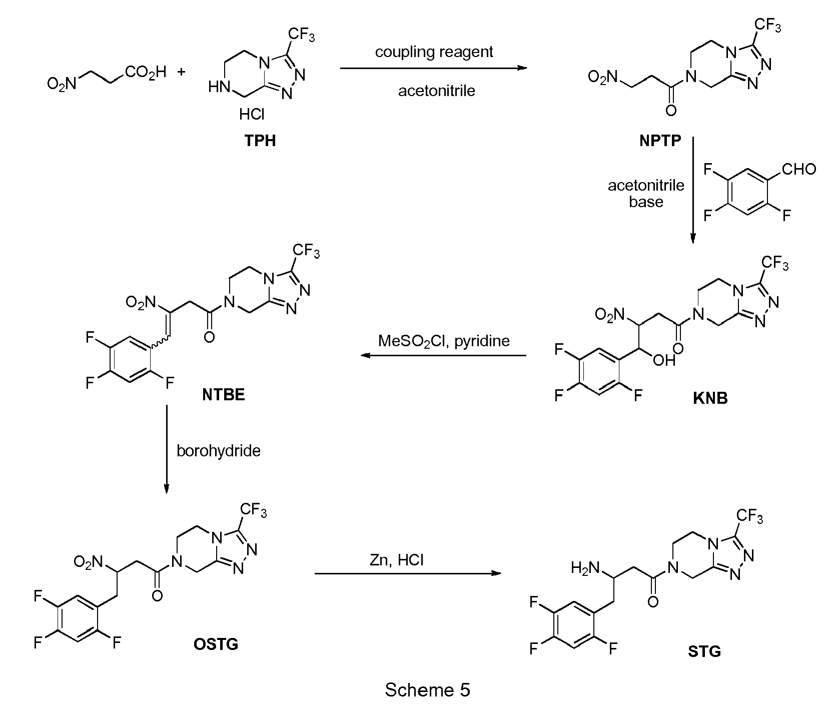

In the first step of the embodiment 3-nitropropionic acid is coupled with **TPH** (compound (IIIa) with R=CF₃, Y=N) in an aprotic solvent selected from halogenated aliphatic hydrocarbons, aromatic hydrocarbons, ethers or nitriles or mixture thereof, preferably in acetonitrile by means of coupling reagents such as thionyl chloride, oxalyl chloride, activated isoureas, or carbodiimides, preferably N-(3-dimethylaminopropyl)-N'-ethylcarbodiimide hydrochloride in 0 - 50 %, preferably 5 - 20 % molar excess, optionally in the presence of bases preferably selected from tertiary amines, such as, triethylamine, ethyldiisopropylamine or N-methylmorpholine to give **NPTP** (compound of formula (IV) with R₁₀=CF₃, Y=N), which is isolated after reducing of volume of solvent, extraction from alkaline water / water immiscible solvent biphasic system and precipitation of concentrated organic solvent solution with ethers, preferably methyl t-butyl ether.

In the next step of the embodiment **NPTP** is coupled with 2,4,5-trifluorobenzaldehyde (compound of formula (V) with (X)ₙ being 2,4,5-trifluoro) in the presence of a base in a polar protic or aprotic solvent, selected from water, alcohols, amides, sulfoxides, sulphones or nitriles, preferably in dry acetonitrile at 0 - 50 °C, preferably at room temperature for 5 - 48 h, preferably for 15 - 25 h. Any base selected from amides, hydrides, hydroxides, amidines, tertiary amines can be used, but the preferred base is selected from inorganic basic salts, such as acetates, carbonates, phosphates, most preferably potassium phosphate tribasic in catalytic submolar amounts, preferably in 5 - 20 % molar amount is used. The product **KNB** (compound of formula (VI) with (X)ₙ being 2,4,5-trifluoro, R₁₀=CF₃, Y=N) is isolated after reducing the reaction solvent and treating with water/chlorinated solvent mixture as a solid mixture of four isomers.

In the next step of the embodiment **KNB** is treated with a sulfonyl chloride or anhydride selected from methanesulfonyl chloride, p-toluenesulfonyl chloride or trifluoromethanesulfonyl anhydride, preferably with methanesulfonyl chloride in 1-3 fold molar excess in the presence of a base selected from tertiary amines or basic heterocycles, preferably in the presence of pyridine. The reaction is carried out in an inert solvent selected from nitriles, esters or chlorinated hydrocarbons, aromatic hydrocarbons, or without solvent in the presence of base in high excess, preferably in pyridine at 0 - 50 °C, preferably at room temperature for 1-10 h. The product **NTBE** (compound of formula (VIII) with (X)ₙ being 2,4,5-trifluoro, R₁₀=CF₃, Y=N) is isolated after purification as a solid powder in (E) and/or (Z) configuration.

In the next step of the embodiment **NTBE** is reduced by catalytic hydrogenation or in the presence of hydrides, preferably it is reduced by aluminium or boron hydrides. In a most preferred example sodium borohydride in C₁-C₄-alcohol, preferably isopropanol is used and the reaction is carried out at 0 - 50 °C, preferably at room temperature for 1 - 10 h. The racemic product **OSTG** (compound of formula (IX) with (X)ₙ being 2,4,5-trifluoro, R₁₀=CF₃, Y=N) is isolated after the removal of inorganic salt by water/organic solvent extraction and chemical purification as a solid powder, which can be separated by preparative chiral chromatography to pure (*R*) and (*S*)-enantiomer of OSTG.

In the last step of this embodiment the nitro group in **OSTG** is reduced. The preferred methods use cheap inorganic reducing agents selected from inorganic sulfides, low-valent metal salts or elemental metal in the presence of acids. The most preferred method of transformation of **OSTG** to **STG** (compound of formula (I) with (X)ₙ being 2,4,5-trifluoro, R₁₀=CF₃, Y=N) is a reduction with zinc and hydrochloric acid in C₁-C₄-alcohol, preferably methanol at 0 - 50 °C, preferably at room temperature for 15 - 120 min, preferably for 20 - 40 min.

In an analogous way (*R*)-OSTG is reduced to sitagliptin (compound (*R*)-STG), which is isolated as oil that slowly solidifies. Sitagliptin can then be further transformed to a pharmaceutically acceptable salt, preferably to phosphate, for example by treating an alcohol solution of sitagliptin base with the corresponding acid of the desired salt, such as with ortho-phosphoric acid.

Alternative and variable synthetic ways to obtain other β-amino acid derivatives and in particular other β-aminobutyryl compounds having γ-phenyl and/or heterocyclic structural moieties become apparent from the description of the embodiments and illustrations above.

In a further aspect the present invention provides key intermediate compounds useful for synthesizing gliptins (dipeptidyl peptidase-4 (DPP-4) inhibitors) as particular β-aminobutyryl compounds having γ-phenyl and/or heterocyclic structural moieties.

In another aspect, the present invention provides a pharmaceutical composition for administering a therapeutically effective amount of a gliptin compound, notably sitagliptin and more preferably its phosphate salt, as produced according to the present invention in unit dosage form with one or more pharmaceutically acceptable carriers or other excipients. A therapeutically effective amount of sitagliptin salt of the present invention is amount of salt ranging, when calculated as sitagliptin base, from 5 to 200 mg, preferably from 10 to 150 mg, more preferably from 25 to 100 mg.

Sitagliptin, preferably its phosphate salt, prepared according to the present invention can be embodied for example in form of tablet, capsules, pellets, granules and suppositories or their combined forms. Pharmaceutical composition in accordance with present invention can be suitable for immediate release or modified release of sitagliptin salts of the present invention. Solid pharmaceutical compositions can be for example coated with aim of increasing peletibility or regulating the disintegration or absorption.

Pharmaceutically acceptable excipients may be selected from the group consisting of binders, diluents, disintegrating agents, stabilizing agents, preservatives, lubricants, fragrances, flavoring agents, sweeteners and other excipients known in the field of the pharmaceutical technology. Preferably, carriers and excipients may be selected from the group consisting of lactose, microcrystalline cellulose, cellulose derivatives, (e.g. hydroxypropylcellulose, croscarmellose sodium), polyacrylates, calcium carbonate, starch, colloidal silicone dioxide, anhydrous dibasic calcium phosphate, sodium starch glycolate, talc, magnesium stearate, sodium stearyl fumarate, mannitol, polyvinylpyrrolidone, polyethylene glycol and other excipients known in the field of the pharmaceutical technology.

Optionally, the pharmaceutical compositions of the invention may be combination products comprising one or more additional pharmaceutically active components in addition to sitagliptin phosphate according to the present invention, preferably one or more additional pharmaceutically active components are selected from the group consisting of insulin sensitizers, insulin, insulin mimetics, sulfonylureas, α-glucosidase inhibitors, glucagon receptor antagonists, GLP-1, GLP-1 analogues, GLP-1 mimetics, GLP-1 receptor agonists, GIP, GIP mimetics, PACAP, PACAP mimetics, PACAP receptor agonists, cholesterol lowering agents, PPARδ agonists, antiobesity compounds, ileal bile acid tranporter inhibitors, agents intended for use in inflammatory conditions, antihypertensive agents, glucokinase activators (GKAs), inhibitors of 11β-hydroxysteroid dehydrogenase type 1, inhibitors of cholesteryl ester transfer protein (CETP) and inhibitors of fructose 1,6-bisphosphatase.

Most preferably additional pharmaceutically active component is metformin and/or its pharmaceutically acceptable salt.

The pharmaceutical compositions according to the present invention may be prepared by methods known in the field of the pharmaceutical technology.

A further aspect of the present invention is a method for treatment and/or prevention in mammal of clinical conditions for which DPP-IV inhibitor is indicated, in particular treatment of Type 2 diabetes, hyperglycemia, insulin resistance, and obesity, with a medicament by using an effective amount of sitagliptin phosphate according to the present invention.

Another aspect the present invention is related to use of sitagliptin, preferably its phosphate salt, prepared according to the present invention for the manufacture of medicament for treatment and/or prevention in mammal of clinical conditions for which DPP-IV inhibitor is indicated, in particular treatment of type 2 diabetes, hyperglycemia, insulin resistance, and obesity.

The following examples illustrate the present invention and are not intended to limit the scope of the invention.

### Example 1: 3-nitro-1-(3-(trifluoromethyl)-5,6-dihydro-[1,2,4]triazolo[4,3-a]pyrazin-7(8H)-yl)propan-1-one (NPTP)

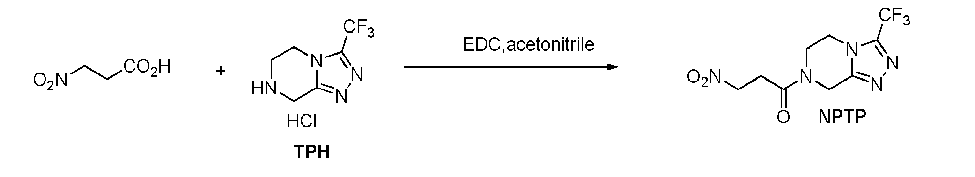

The title compound was prepared using the method described in WO 2008/040974.

A mixture of 3-nitropropionic acid (20.0 g, 0.168 mol), 3-(trifluoromethyl)-5,6,7,8-tetrahydro-[1,2,4]triazolo[4,3-a]pyrazine hydrochloride (48.0 g, 0.21 mol) in acetonitrile (400 mL) is cooled to 0 °C and 4-methylmorpholine (16.9 g, 0.168 mmol) is added, followed by N-(3-dimethylaminopropyl)-N'-ethylcarbodiimide hydrochloride (48.4 g, 0.25 mol) after 5 min. The resulting mixture is stirred at room temperature for 20 hours. The resulting mixture is concentrated to about 2/3 of it volume and ethyl acetate is added (750 mL). The resulting mixture is washed twice with water (200 + 100 mL), sat. aq. sodium hydrogencarbonate (200 mL), brine (200 mL) and dried with sodium sulfate. The resulting clear solution is concentrated under reduced volume and MTBE is added (100 mL). The precipitated white solid is filtered off, washed with MTBE (200 mL) and dried under reduced pressure to yield 38.8 g (79 % yield) of **NTPT** as white powder.

### Example 2: 4-hydroxy-3-nitro-1-(3-(trifluoromethyl)-5,6-dihydro-[1,2,4]triazolo[4,3-a]pyrazin-7(8H)-yl)-4-(2,4,5-trifluorophenyl)butan-1-one (KNB)

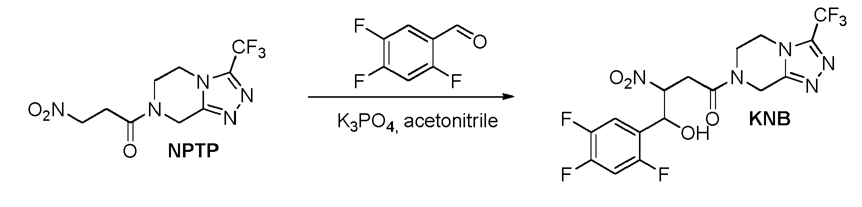

To a mixture of **NPTP** (29.3 g, 100 mmol), potassium phosphate tribasic (1.54 g, 7.3 mmol) and dry acetonitrile (135 mL) is added 2,4,5-trifluorobenzaldehyde (11.4 mL, 100 mmol) and the resulting mixture is stirred at room temperature for 20 hours. The resulting mixture is concentrated under reduced pressure and water (500 mL) and dichloromethane (50 mL) are added. The mixture is cooled to 0 °C and stirred at this temperature for 1 hour. During this period a white precipitate is formed, which is filtered off and washed with cold water (100 mL) followed by cold dichloromethane (100 mL) to yield 30.9 g (68 % yield) of **KNB** as white powder. From filtrate another fraction precipitates out, which is also filtered off and washed with cold water (100 mL) followed by cold dichloromethane (100 mL) to yield 4.1g (9 % yield) of **KNB** as white powder. Both fractions are combined to yield a total of 35 g (77 % yield) of **KNB** as mixture of diastereoisomers. First diastereoisomer: ¹H NMR (DMSO-D6): δ 2.67 (m, 1H), 3.47 (m, 1H), 3.70 (m, 0.5H), 3.90-4.27 (m, 3.5H), 4.66-5.15 (m, 3H), 5.49 (s, 1H), 6.62 (m, 1H), 7.51-7.70 (m, 2H). Second diastereoisomer: ¹H NMR (DMSO-D6): δ 3.01 (m, 1H), 3.20 (m, 0.5H), 3.44 (m, 1H), 3.80 (m, 0.5H), 3.88-4.32 (m, 4H), 4.74 (m, 1H), 4.86-5.02 (m, 2H), 5.11-5.28 (m, 2H), 6.56 (m, 1H), 7.47-7.62 (m, 2H). HPLC-MS (ES, m/z): 454 [M+H]⁺,

### Example 3: 3-nitro-1-(3-(trifluoromethyl)-5,6-dihydro-[1,2,4]triazolo[4,3-a]pyrazin-7(8H)-yl)-4-(2,4,5-trifluorophenyl)but-3-en-1-one (NTBE)

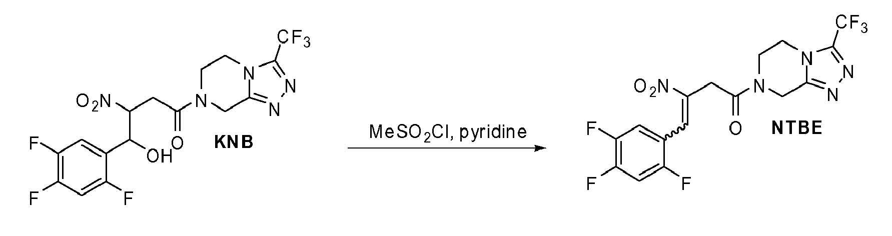

To a solution of **KNB** (45.3 g, 100 mmol) in pyridine (200 mL) is added methanesulfonyl chloride (25.4 g, 222 mmol) and the resulting mixture is stirred at room temperature for 4 hours. The solution is cooled to 0 °C and water (5 mL) is added, followed by Celite (100 g). Pyridine is removed under reduced pressure and the resulting mixture is subjected to chromatography (silica gel; hexane : ethyl acetate = 50 : 50 → 0 : 100) to give 29.5 g (68 % yield) of NTBE as yellowish powder. ¹H NMR (DMSO-D6): δ 3.98 (m, 1H), 4.05-4.17 (m, 4H), 4.28 (m, 1H), 4.92 (s, 1H), 5.12 (s, 1H), 7.63-7.79 (m, 2H), 7.10 (m, 1H). HPLC-MS (ES, m/z): 436 [M+H]⁺.

### Example 4 : 3-nitro-1-(3-(trifluoromethyl)-5,6-dihydro-[1,2,4]triazolo[4,3-a]pyrazin-7(8H)-yl)-4-(2,4,5-trifluorophenyl)but-3-en-1-one (NTBE)

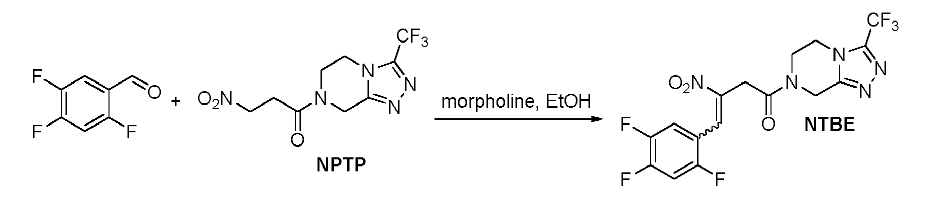

A mixture of **NPTP** (1.17 g, 4 mmol), 2,4,5-trifluorobenzaldehyde (0.64 g, 4 mmol), morpholine (0.20 mL, 2.3 mmol) and ethanol (4 mL) is refluxed for 3h and concentrated. The resulting oil is subjected to chromatography (silica gel; toluene : ethyl acetate = 80 : 20 → 50 : 50) to give 0.25 g (14 % yield) of NTBE as yellow powder.

### Example 5: 3-nitro-1-(3-(trifluoromethyl)-5,6-dihydro-[1,2,4]triazolo[4,3-a]pyrazin-7(8H)-yl)-4-(2,4,5-trifluorophenyl)butan-1-one (OSTG)

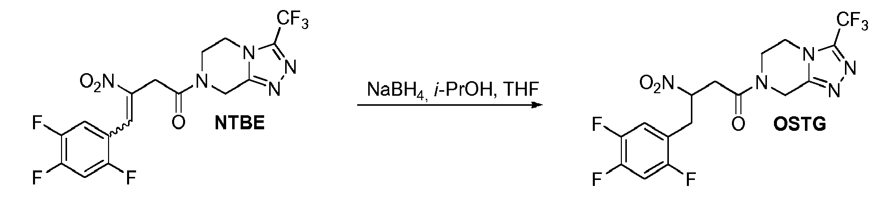

To a cold (0 °C) solution of **NTBE** (2.17 g, 5 mmol) in a mixture of 2-propanol (5 mL) and tetrahydrofurane (10 mL) is added sodium borohydride (227 mg, 6 mmol) and the resulting mixture is stirred for 3 hours. To the resulting mixture is added 40 % aqueous phosphoric acid (5 mL) and water. The organic solvents are removed under reduced pressure and dichloromethane (20 mL) is added, followed by 1 M aqueous sodium hydroxide so that the pH is 8. The organic phase is dried with sodium sulfate and concentrated under reduced pressure. The resulting white powder is washed with a mixture of ethyl acetate/hexane 1:1 (v/v) (25 mL) and dried under reduced pressure to give 0.84 g (39 % yield) of racemic **OSTG** as white powder. ¹H NMR (DMSO-D6): δ 3.07-3.28 (m, 3H), 3.35-3.47 (m, 1H), 3.78-4.19 (m, 3H), 4.21-4.34 (m, 1H), 4.71-5.18 (m, 3H), 7.47-7.62 (m, 2H). HPLC-MS (ES, m/z): 438 [M+H]⁺.

**OSTG** is optionally further separated by chiral column chromatography to 0.36 g of (*R*)-**OSTG** and 0.31 g of *(S)-***OSTG** after evaporation of the mobile phase as white powders.

### Example 6: 3-amino-1-(3-(trifluoromethyl)-5,6-dihydro-[1,2,4]triazolo[4,3-a]pyrazin-7(8H)-yl)-4-(2,4,5-trifluorophenyl)butan-1-one (STG)

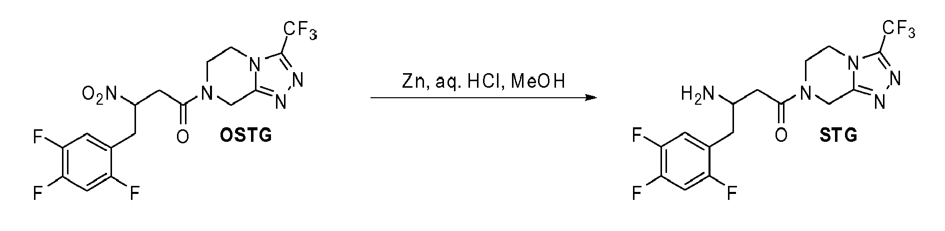

To a mixture of OSTG (0.11 g, 0.25 mmol), powdered zinc (0.12 g) and methanol (2 mL) is added drop wise a 37 % aqueous HCl and the mixture is stirred at room temperature for 30 min. Zinc is removed by filtration and the resulting clear solution is concentrated under reduced pressure. To the remaining oil is added water (5 mL), followed by 1 M aqueous sodium hydroxide so that the pH is 10 and the product is extracted with dichloromethane (3 x 10 mL). The organic phases are combined, dried with sodium sulfate and concentrated under reduced pressure to give 95 mg (93 % yield) of **STG** as yellowish oil.

### Example 7: Sitagliptin phosphate

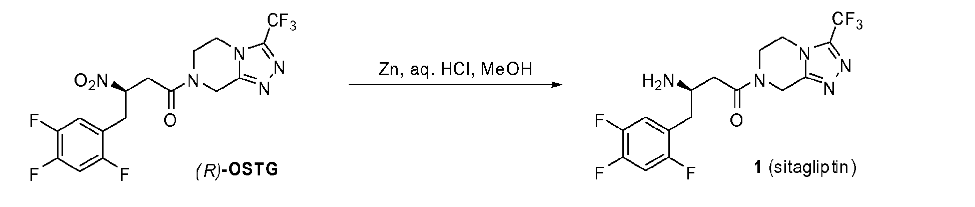

To a mixture of (*R*)-**OSTG** (0.11 g, 0.25 mmol), powdered zinc (0.12 g) and methanol (2 ml) is added drop wise a 37 % aqueous HCl and the mixture is stirred at room temperature for 30 min. Zinc is removed by filtration and the resulting clear solution is concentrated under reduced pressure. To the remaining oil is added water (5 ml), followed by 1 M aqueous sodium hydroxide so that the pH is 10 and the product is extracted with dichloromethane (3 x 10 ml). The organic phases are combined, dried with sodium sulfate and concentrated under reduced pressure to give 89 mg (87 % yield) of sitagliptin base as glassy solid.

The free base of 1 is dissolved in 1 ml of ethanol followed by the addition of 0.05 g 85% ortho-phosphoric acid. The resulting suspension is cooled for 15 min at 0 °C and filtered. The product is washed with 1 ml of ethanol and 2.5 ml of ether and dried on filter for 1 h. 85 mg of the off-white powdery product of sitagliptin phosphate is obtained.

## Claims

1. A process for the preparation of a β-amino amide compound, comprising:
(i) providing a 2-nitropropionic acid derivative of formula (IIc) 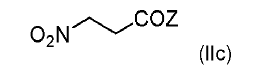 wherein Z is
(i-1) a substituent defined by the following compound of formula (III') 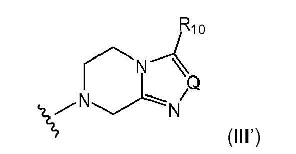 wherein Q is N, CH, C-CF₃, or C-phenyl, preferably N, and R₁₀ is H, C₁-C₄-alkyl or fluorinated C₁-C₂-alkyl, preferably trifluoromethyl,
or
(i-2) OH, OR¹, NR²R³ or OSiR⁴R⁵R⁶, wherein R¹ is C₁-C₆-alkyl or aryl-C₁-C₂-alkyl, R² is H, C₁-C₄-alkyl, arylmethyl, C₁-C₄-alkoxy or arylmethoxy, R³ is H, C₁-C₄-alkyl or arylmethyl, and R⁴, R⁵ and R⁶ independently are C₁-C₄-alkyl or phenyl,
(ii) carrying out a coupling reaction (condensation) of the 2-nitropropionic acid derivative of formula (IIc) with an aldehyde of formula Ar-CHO, preferably in the presence of a base substance, wherein the aldehyde Ar-CHO is defined by formula (V) 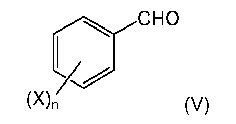 wherein X is halogen selected from fluoro, chloro, or bromo, preferably fluoro, same or different, and n is 1-4,
and obtaining a compound of formula (VIIIa) or (VIa) 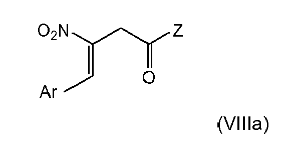 or 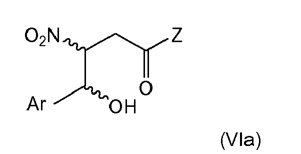
(iii-1) subjecting the compound of formula (VIIIa) or (VIa) to a conversion reaction to obtain a compound of formula (IXa) 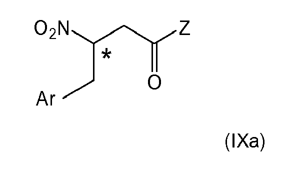 by a reaction selected from the group of esterification, dehydration, reduction, hydrogenation, elimination and combinations thereof,
or
(iii-2) subjecting the compound of formula (VIIIa) to a reduction reaction to obtain 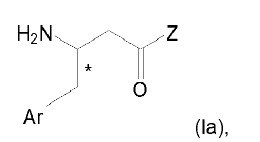 optionally forming a salt thereof;
wherein the stereogenic center marked with an * is either in (*R*)- or (*S*)-configuration at marked center, or it is in racemic form,
(iv) if Z optionally is as defined in (i-2), subjecting Z to structural modification,
wherein the structural modification comprises steps of
• converting a compound of formula (XIIIa) 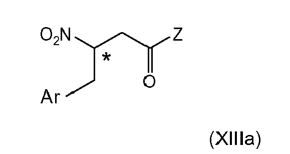 (if Z is other than OH) to compound of formula (XIVa), 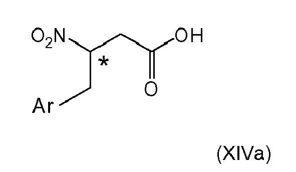
• activating the carboxylic group in the compound of formula (XIVa) and converting the activated derivative of formula (XIVa) to compound of formula (IXb) 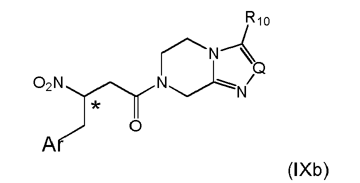 by reacting with compound of formula (III) 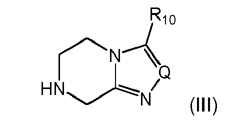 wherein Ar, * , Q and R₁₀ are as defined above
yielding a β-nitro amide compound;
(v) if the process optionally proceeds via (iii-1) or (iv), reducing the nitro group in compound of formula (IXa) or the reaction products of (iv) to an amino group, optionally forming a salt thereof.

2. The process according to claim 1, wherein the step of providing 2-nitropropionic amide derivative of formula (IIc) as defined in (i-1) comprises
activating 3-nitropropionic acid of formula (II) 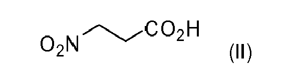 by conversion to
(i) halogenide of formula (IIa) 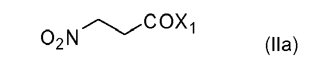 wherein X₁ is chloro, bromo, preferably chloro, by reaction with a sulfur, phosphorous, oxalyl or phosgene halogen derivative;
(ii) or anhydride of formula (IIb) 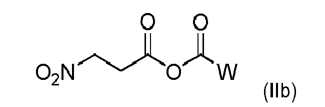 wherein W is selected from the group consisting of C₁-C₆-alkyl residues, C₁-C₆-alkoxy residues, unsubstituted or substituted benzyloxy, by reaction with acyl chlorides, preferably pivaloyl chloride, or chlorofomates;
(iii) or *in situ* formed intermediate by reaction with a coupling reagent known from peptide chemistry, selected from the group consisting of
carbodiimides, preferably N,N'-dicyclohexylcarbodiimide (DCC), N,N'-diisopropylcarbodiimide (DPCI), or N-ethyl-N'-(3-dimethyaminopropyl) carbodiimide (EDC), with or without 1-hydroxybenzotriazole;
activated ureas, preferably O-(benzotriazol-1-yl)-N,N,N',N'-tetramethyluronium hexafluorophosphate (HBTU), O-(benzotriazol-1-yl)-N,N,N',N'-tetramethyluronium tetrafluoroborate (TBTU), and O-(7-aza-benzotriazol-1-yl)-N,N,N',N'-tetramethyl uronium hexafluorophosphate (HATU);
carbonyldiimidazole; and
1-methylpyridinium iodide; and
reacting the activated derivative of 3-nitropropanoic acid with the compound of formula (III) 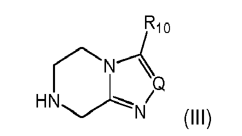 wherein Q and R₁₀ are as defined above.

3. The process according to claims 1 or 2, wherein the compound of formula (VIa) is obtained and subjected to a dehydration reaction by treating said compound with a strong acid or by using a dehydrating agent, respectively to give a compound of formula (VIIIa), 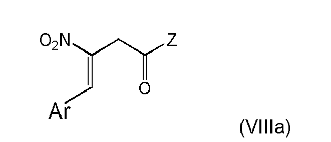 wherein Ar and Z are as defined above.

4. The process according to any one of the preceding claims, wherein the compound of formula (VIa) is subjected to a reduction reaction to reduce the benzylic hydroxyl group by treatment with silicon hydrides, or by catalytic hydrogenation in the presence of a precious metal catalyst, or by transformation to an ester and catalytic hydrogenation of the ester, respectively to give the compound of formula (IXa), 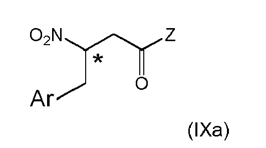 wherein the stereogenic center marked with an * is either in (*R*)- or (*S*)-configuration at marked center, or it is in racemic form; and Ar and Z are as defined above.

5. The process according to any one of the preceding claims, wherein the compound of formula (VIa) is subjected to an esterification reaction by halogenides or anhydrides of unsubstituted or halogenated C₁-C₆-alkanoic or C₁-C₆-alkanesulfonic acids, unsubstituted or para substituted benzenesulfonic acids, respectively in the presence of a base substance to give an ester compound of formula (VIIa), 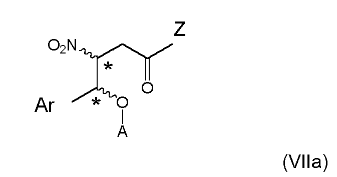 wherein stereogenic centers are marked with an *; these can be either in (*R*)- or (*S*)-configuration at marked center; Ar and Z are as defined above, and A is C₁-C₆-alkanoyl, halogenated C₁-C₆-alkanoyl, C₁-C₆-alkanesulfonyl, unsubstituted or para substituted benzenesulfonyl.

6. The process according to claim 5, wherein the ester compound of formula (VIIa) is subjected to base-induced or thermal elimination of AOH from said compound to give a compound of formula (VIIIa)

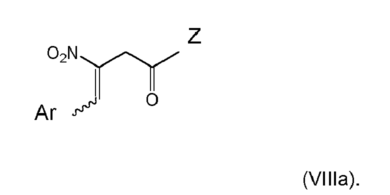

7. The process according to any one of claims 3 and 6, wherein the double bond of the compound of formula (VIIIa) is reduced by a reducing agent, by catalytic hydrogenation or by biotransformation, respectively to give a compound of formula (IXa), 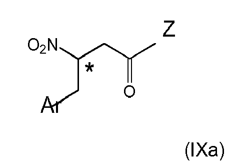 wherein the stereogenic center marked with an * is either in (*R*)- or (*S*)-configuration at marked center, or it is in racemic form, and Ar is as defined above.

8. The process according to any one of claims 1, 3 and 6, wherein the compound of formula (VIIIa) is subjected to a simultaneous reduction of the double bond and the nitro group by a suitable reduction system to give the compound of formula (Ia), 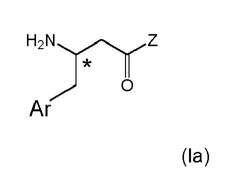 wherein the stereogenic center marked with an * is either in (*R*)- or (*S*)-configuration at marked center, or it is in racemic form, Ar and Z are as defined above.

9. The process according to any one of the claims 1 to 7, wherein in (v) the nitro group is converted into an amino group by contacting with a reducing agent, preferably selected from elemental metals in the presence of acids, preferably zinc and tin, cations in low oxidation states selected from Fe(II), Sn(II), Cr (II), hydrogen sulfide, metal sulfides and polysulfides,
or by catalytic hydrogenation in the presence of a metal catalyst, preferably selected from nickel, palladium, rhodium, iridium, gold and platinum, to give the final compound of formula (I).

10. The process according to any one of claims 1 and 3 to 9, wherein when the 2-nitropropionic acid derivative of formula (IIc) 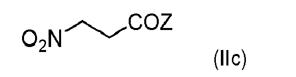 is provided with Z being defined as in claim 1, (i-2)
the coupling reaction (ii) with an aldehyde of formula (V) is carried out forming the intermediates of formula (Xa) or (XIIa), 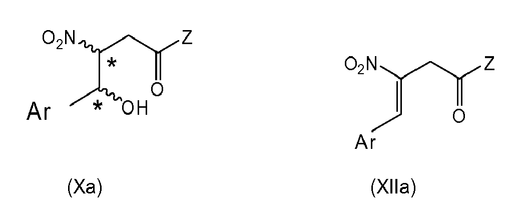 wherein Ar and * are as defined above,
and wherein subsequently the intermediates of formula (Xa), or (XIIa) are subjected to the conversion (iii) to obtain a compound of formula (XIIIa), 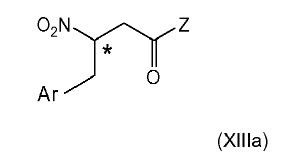 wherein compounds of formulae (Xa), (Xlla) and (Xllla) are respective structural analogues of compounds of formulae (VIa), (VIIIa) and (IXa), in which Z is defined as in item 1, (i-2) and
wherein the compound of formula (XIIIa), if Z is other than OH, is converted by a method selected from the group consisting of hydrolysis, reduction and hydrogenation, to give the respective carboxylic compound formula (XIVa) 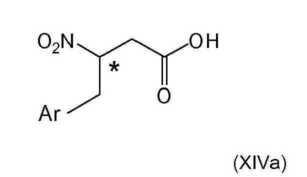 which is subjected to further structural modification.

11. The process according to claim 10, wherein subjecting compound of formula (XIVa) to further structural modification involves converting the carboxylic group to an activated carboxylic group by
(i) reaction with a sulfur, phosphorous, oxalyl or phosgene halogen derivative to obtain respective halogenide COX₁, wherein X₁ is chloro, bromo, preferably chloro; or
(ii) reaction with acyl chlorides, preferably pivaloyl chloride, or chlorofomates to obtain an anhydride wherein Z is defined below 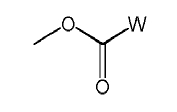 wherein W is C₁-C₆-alkyl or C₁-C₆-alkoxy, unsubstituted or substituted benzyloxy; or
(iii) by reaction with coupling reagents which are ordinary used in peptide chemistry to obtain *in situ* formed intermediates, wherein the coupling reagents are selected from the group consisting of:
carbodiimides, preferably from N,N'-dicyclohexylcarbodiimide (DCC), N,N'-diisopropylcarbodiimide (DPCI), or N-ethyl-N'-(3-dimethyaminopropyl) carbodiimide (EDC), with or without 1-hydroxybenzotriazole,
activated ureas preferably O-(benzotriazol-1-yl)-N,N,N',N'-tetramethyluronium hexafluorophosphate (HBTU), O-(benzotriazol-1-yl)-N,N,N',N'-tetramethyluronium tetrafluoroborate (TBTU), and O-(7-aza-benzotriazol-1-yl)-N,N,N',N'-tetramethyl uronium hexafluorophosphate (HATU),
carbonyldiimidazole,
1-methylpyridinium iodide.

12. The process according to claim 11, wherein the activated compound obtained in either one of (i), (ii) or (iii) is reacted with a compound of formula (III) 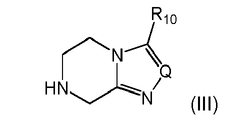 wherein Q is N, CH, C-CF₃, or C-phenyl, preferably N, and R₁₀ is H, C₁-C₄-alkyl or fluorinated C₁-C₂-alkyl, preferably trifluoromethyl.

13. The process according to any one of claims 1 to 6 and 9 to 12, wherein the compound of formula (VIIIa) is subjected to hydrogenation in the presence of a transition metal and at least one chiral ligand to give a compound of formula (IXa) 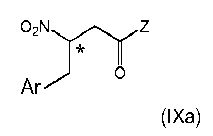 wherein Ar and Z are as defined above,
wherein the configuration on stereogenic center marked with an * is enriched in (*R*)- or (*S*)-configuration, preferably is enriched in (*R*)-enantiomer.

14. The process according to any one of the preceding claims, wherein the compound of formula (VIIIa) or (XIIa) is subjected to reduction by a reductase, selected from reductase enzymes of various microorganisms, preferably selected from reductase enzymes derived from Actinomyces, Saccharomyces (in particular baker's yeast) or Bacteria gens Clostridium.

15. The process according to any one of the preceding claims, wherein the amide moiety is defined by the following formula (III) 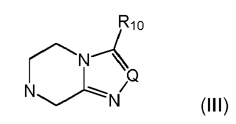 wherein Q is N and R₁₀ is trifluoromethyl.

16. The process according to any one of the preceding claims, wherein the aldehyde Ar-CHO is defined by formula (Va)

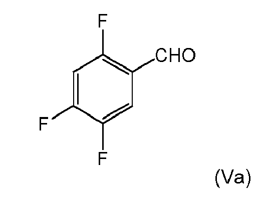

17. A compound defined by any one of the following formulae (VI), (VII), (VIII) and (IX) 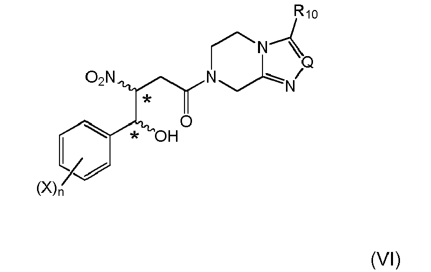 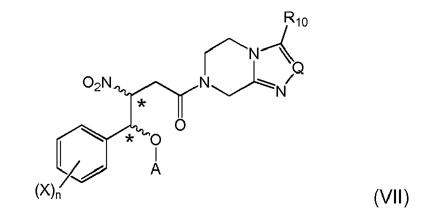 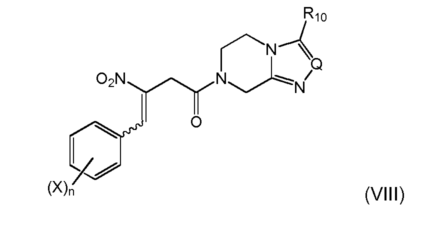 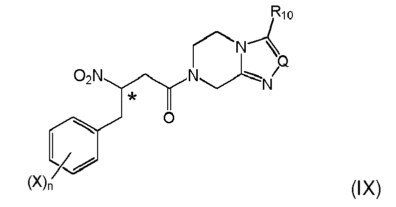 wherein a stereogenic center, if any, is marked with an * and is either in (*R*)- or (*S*)-configuration at marked center, or it is in racemic form, preferably in (*R*)-configuration;
wherein the stereogenic centers marked with an * are either in (*R*)- or (*S*)-configuration at marked center; X is halogen selected from fluoro, chloro, or bromo, preferably fluoro, same or different, and n is 1-4, preferably 3; Q is N, CH, C-CF₃, or C-phenyl, preferably N; and R₁₀ is H, C₁-C₄-alkyl or fluorinated C₁-C₂-alkyl, preferably trifluoromethyl; A is C₁-C₆-alkanoyl, halogenated C₁-C₆-alkanoyl, C₁-C₆-alkanesulfonyl, unsubstituted or para substituted benzenesulfonyl;

18. Use of a compound selected from the compounds of formulae (VI), (VII), (VIII) and (IX) as defined in claim 17 for the manufacture of dipeptidyl peptidase-4 (DPP-4) inhibitors, preferably sitagliptin.

19. Process for the preparation of a pharmaceutical composition comprising a gliptin, comprising:
carrying out a process according to any one of claims 1 to 18 and obtaining a gliptin compound;
optionally transforming said gliptin compound into its pharmaceutically acceptable salt, preferably its phosphate salt;
formulating said gliptin compound or its pharmaceutically acceptable salt with at least one pharmaceutical excipient.

20. The process according to claim 19, wherein said pharmaceutical composition comprises a combination of said gliptin compound and one or more additional pharmaceutically active ingredient(s), preferably selected from the group consisting of insulin sensitizers, insulin, insulin mimetics, sulfonylureas, α-glucosidase inhibitors, glucagon receptor antagonists, GLP-1, GLP-1 analogues, GLP-1 mimetics, GLP-1 receptor agonists, GIP, GIP mimetics, PACAP, PACAP mimetics, PACAP receptor agonists, cholesterol lowering agents, PPARδ agonists, antiobesity compounds, ileal bile acid tranporter inhibitors, agents intended for use in inflammatory conditions, antihypertensive agents, glucokinase activators (GKAs), inhibitors of 11β-hydroxysteroid dehydrogenase type 1, inhibitors of cholesteryl ester transfer protein (CETP) and inhibitors of fructose 1,6-bisphosphatase; preferably said additional pharmaceutically active ingredient is metformin and/or its pharmaceutically acceptable salt.
